# EUROPEAN PATENT SPECIFICATION

(11) **EP 2 707 395 B1**
(45) Date of publication and mention of the grant of the patent: **12.04.2017**
(21) Application number: 12786737.2
(22) Date of filing: 11.05.2012
(51) Int. Cl.: C07K 19/00, C07K 14/705, G01N 33/68

(54) **NOVEL FUSION PARTNERS FOR THE PURPOSE OF CRYSTALLIZING G-PROTEIN COUPLED RECEPTORS**
NEUE FUSIONSPARTNER ZUR KRISTALLISIERUNG G-PROTEIN-GEKOPPELTER REZEPTOREN
PARTENAIRES DE FUSION INÉDITS UTILISABLES EN VUE DE LA CRISTALLISATION DES RÉCEPTEURS COUPLÉS AUX PROTÉINES G

(30) Priority: 13.05.2011 US 201161485872 P; 30.03.2012 US 201261618424 P
(43) Date of publication of application: 19.03.2014
(73) Proprietor: Receptos, Inc., San Diego, CA 92121 (US); The Scripps Research Institute, La Jolla, CA 92037 (US)
(72) Inventor: HANSON, Michael, A., San Marcos, CA 92078 (US); ROTH, Christopher, B., San Diego, CA 92101 (US); STEVENS, Raymond, C., La Jolla, CA 92037 (US); KUNKEN, Joshua, M., San Diego, CA 92121-2026 (US); GRIFFITH, Mark, T., San Diego, CA 92115 (US); THOMPSON, Aaron, A., San Diego, CA 92117 (US); LIU, Wei, San Diego, CA 92122 (US); XU, Fei, Palm Beach Gardens, FL 33400 (US); KATRITCH, Vsevolod, San Diego, CA 92130 (US)
(74) Representative: Cohausz & Florack
(86) International application number: PCT/US2012/037622
(87) International publication number: WO 2012/158555

(56) References cited:
- US-A1- 2006 128 944
- US-A1- 2011 009 603
- US-A1- 2012 100 593
- ISHIHARA G ET AL: "Expression of G protein coupled receptors in a cell-free translational system using detergents and thioredoxin-fusion vectors", 1 May 2005 (2005-05-01), PROTEIN EXPRESSION AND PURIFICATION, ACADEMIC PRESS, SAN DIEGO, CA, PAGE(S) 27 - 37, XP027429979, ISSN: 1046-5928 [retrieved on 2005-03-28] * page 30, right-hand column, paragraph 2 *
- HARDING P J ET AL: "Neurotensin receptor type 1: Escherichia coli expression, purification, characterization and biophysical study", August 2007 (2007-08), BIOCHEMICAL SOCIETY TRANSACTIONS, VOL. 35, NR. PART 4, PAGE(S) 760-763, XP002742626, ISSN: 0300-5127 * page 761, right-hand column, paragraph 3 *
- VADIM CHEREZOV ET AL.: 'High Resolution Crystal Structure of an Engineered Human Beta 2-Adrenergic G protein-Coupled Receptor' SCIENCE vol. 318, November 2007, pages 1258 - 1265, XP002487477
- DANIEL M. ROSENBAUM ET AL.: 'GPCR Engineering Yields High-Resolution Structural Insights into Beta2-Adrenergic Receptor Function' SCIENCE vol. 318, November 2007, pages 1266 - 1273, XP002601380

## Description

### BACKGROUND OF THE INVENTION

### Field of the Invention

Fusion partner proteins comprising G protein coupled receptors (GPCRs) having a fusion partner in substitution for a portion of an intracellular domain, or terminally attached at an N or C terminus, of the GPCR where such fusion partner protein is amenable to the formation of diffraction quality crystals to support structure determination for such GPCR.

### Description of Related Art

G-protein coupled receptors comprise a broad class of membrane-bound proteins that share a variety of structural and functional attributes (Friedricksson et al. Mol. Pharmacol. 63(6): 1256-1272, 2003; and Friedricksson et al., Mol. Pharmacol. 67(5): 1414-1425, 2005). GPCRs are classified into 1 of 6 classes: A, B, C, D, E, and F, see Friedricksson *et al.* (2003) and Friedricksson *et al.* (2005). GPCRs comprise seven transmembrane helical regions, as well as an extracellular portion that binds endogenous ligands. This extracellular ligand binding domain is frequently a target site for pharmaceutical agents that modulate GPCR function.

Biochemical Society Transactions, Volume 35, part 4, 2007, p. 760-763 describes the expression of rat NTS1 in *E. coli* in an active ligand-binding form at the cell membrane and the purification for structural biology studies. Ligand-binding activity was determined using both conventional radioligand binding assays and application of SPR (surface plasmon resonance).

US 2011/0009603 A describes a method for crystallizing a GPCR. In this method a fusion protein is employed that comprises a first portion of a G-protein coupled receptor (GPCR), where the first portion comprises the TM1, TM2, TM3, TM4 and TM5 regions of the GPCR, a folded protein insertion, and a second portion of the GPCR, where the second portion comprises the TM6 and TM7 regions of the GPCR. US 2011/0009603 A also describes that the folded protein insertion may be wild-type lysozymes and variants thereof including lysozymes from phage T4.

Protein Expression and Purification, Volume 41, May 2005, p. 27-31 describes a cell-free protein expression with an *E. coli* S30 extract, with G protein coupled receptors as the target integral membrane proteins. In this system, die thioredoxin-fusion vector induced high protein expression levels as compared with the non-fusion and hexa-histidine-tagged proteins. Two detergents, Brij35 and digitonin, solubilized the produced GPCRs, with little or no effect on the protein yields.

Crystallization of proteins can be performed to obtain crystal structures that allow high-resolution structural analysis to produce an accurate three-dimensional structure of the protein, such as an active site or ligand-binding site, where such structural information can then be utilized to predict functionality and behavior of the protein. Crystallization and structure determination by X-ray crystallography requires well-diffracting crystals that can be consistently grown from highly purified and concentrated receptor that is stable in crystallization screens. GPCRs are difficult to crystallize in an unmodified form as GPCRs are often unstable in the detergent micelles used for purification, and may lack sufficient protein surface area available for the formation of crystal contacts.

The incorporation of a fusion partner into a protein has been used to support crystallization of proteins that are difficult to crystallize in unmodified form. (Engel; et al., "Insertion of carrier proteins into hydrophilic loops of the Escherichia coli lactose permease," Biochemica et Biophysica Acta (2002), 1564:38-46; Prive, "Fusion proteins as Tools for Crystallization: the Lactose Permease from Escherichia coli," Acta Cryst. (1994), D50:375-379; Prive; et al., "Engineering the Lac Permease for Purification and Crystallization," Journal of Bioenergetics and Biomembranes (1996), 28(1):29-34).

T4 lysozyme (T4L) has been used as a fusion partner in a GPCR fusion protein that retains desired biochemical, pharmacologic, and structure properties, and can be crystallized. T4L was incorporated as a fusion partner into an available intracellular loop of the β2-adrenergic receptor (β2AR) and the A2A-Adenosine Receptor (A_{2A}), as an aid to receptor stability and crystallization, resulting in structure determinations of resulting GPCR fusion proteins. Inclusion of T4L was found to significantly improve the expression, purification, and crystallization of these two receptors. (Cherezov, V., Rosenbaum, D.M., Hanson, M.A., Rasmussen, S. G., Thian, F. S., Kobilka, T.S., Choi, H.J., Kuhn, P., Weis, W.I., Kobilka, B.K., and Stevens, R.C., "High-resolution crystal structure of an engineered human beta2-adrenergic G protein-coupled receptor," Science 318: 1258-1265, 2007 (Cherezov *et al.*, 2007); Rosenbaum, D.M., Cherezov, V., Hanson, M.A., Rasmussen, S. G., Thian, F. S., Kobilka, T.S., Choi, H.J., Yao, X.J., Weis, W.I., Stevens, R.C., and Kobilka, B.K., "GPCR engineering yields high-resolution structural insights into beta2-adrenergic receptor function," Science 318: 1266-1273, 2007 (Rosenbaum *et al.* 2007); Jaakola, E.P. et al. "The 2.6 angstrom crystal structure of a human A2A adenosine receptor bound to an antagonist," Science 322:1211-1217, 2008 (Jaakola *et al.*, 2008); U.S. Patent No. 7,790,950 B2, PCT Publication WO 2009/055509 A2) To overcome the structural flexibility of, and facilitate crystallization of, the β2AR (a well-studied prototype for GPCRs that respond to diffusible hormones and neurotransmitters), a β2AR fusion protein was engineered in which T4L replaces most of the third intracellular loop of the GPCR, yielding β2AR-T4L that retained near-native pharmacologic properties, and could be crystallized for high-resolution structural analysis. The crystal structure of a human β2AR-T4L bound to the partial inverse agonist carazolol at 2.4 Å resolution was determined, and this structure provided a high-resolution view of a human G protein-coupled receptor bound to a diffusible ligand, which demonstrated that ligand-binding site accessibility is enabled by the second extracellular loop, which is held out of the binding cavity by a pair of closely spaced disulfide bridges and a short helical segment within the loop. (Cherezov et al., 2007, Science 318:1258) Analysis of adrenergic receptor ligand-binding mutants within the context of the reported high-resolution structure of β2ART4L provides insights into inverse-agonist binding and the structural changes required to accommodate catecholamine agonists. (Rosenbaum et al. 2007, Science 318:1266)

### BRIEF SUMMARY OF THE INVENTION

The invention provides a composition comprising a GPCR-fusion partner protein which comprises, from N-terminus to C terminus: (i) a first domain comprising a portion of a G-protein-coupled receptor (GPCR) wherein the first domain comprises the first through fifth transmembrane domains of the GPCR, (ii) a second domain comprising a fusion partner amino acid sequence wherein said sequence is at least 90% homologous to rubredoxin, and (iii) a third domain comprising a portion of the GPCR wherein the third domain comprises the sixth and seventh transmembrane domains of the GPCR.

The invention also concerns a composition comprises a GPCR-fusion partner protein which comprises, from N-terminus to C terminus: (i) a first domain comprising a G-protein-coupled receptor (GPCR), and (ii) a second domain comprising a fusion partner amino acid sequence wherein said sequence is at least 90% homologous to rubredoxin.

The invention further relates to a composition composition comprises a GPCR-fusion partner protein which comprises, from N-terminus to C terminus: (i) a first domain comprising a fusion partner amino acid sequence wherein said sequence is at least 90% homologous to rubredoxin, and (ii) a second domain comprising a GPCR.

The invention also concerns a method of using a fusion partner to support crystallization of a protein suitable for crystallographic structural studies of a G-protein-coupled receptor (GPCR) comprising: (a) incorporating a fusion partner into an intracellular domain of the GPCR to form a GPCR-fusion partner protein, wherein the fusion partner comprises an amino acid sequence which is at least 90% homologous with the amino acid sequence of rubredoxin; (b) expressing and purifying the GPCR-fusion partner protein; and (c) crystallizing the purified GPCR-fusion partner protein.

The invention is also directed to a method that uses a fusion partner to support crystallization of a protein suitable for crystallographic structural studies of a G-protein-coupled receptor (GPCR) comprising: (a) attaching a fusion partner to an N-terminus or C-terminus of the GPCR to form a GPCR-fusion partner protein, wherein
the fusion partner comprises an amino acid sequence which is at least 90% homologous with the amino acid sequence of rubredoxin; (b) expressing and purifying the GPCR-fusion partner protein; and (c)crystallizing the purified GPCR-fusion partner protein.

In certain embodiments the fusion partner is substituted for some or all of the third intracellular loop of the GPCR between the fifth and sixth helix of the GPCR. In certain other embodiments, the fusion partner is attached at the C-terminus or the N-terminus of the GPCR. In certain other embodiments the C or N terminus of the GPCR is truncated and the fusion partner is attached at such truncated terminus. In certain embodiments the fusion partner protein is in crystalline form. In further embodiments such crystals are of a quality suitable for x-ray crystallographic structure determination of the GPCR. In certain embodiments such structure (or a portion thereof) can be obtained with a resolution of between at least 3.5 A to at least 1.5 Å. In certain further non-limiting embodiments, such structure (or a portion thereof) can be obtained with a resolution of at least, 3.5 Å, 3.4 A, 3.3 Å, 3.2 A, 3.1 Å 3.0 Å, 2.9 A, 2.8 Å, 2.7 Å, 2.6 Å, 2.4 A, or 2.4 Å.

In certain embodiments the invention provides a method for the preparation of a GPCR in a crystalline form comprising preparing a nucleotide sequence suitable for the expression of an amino acid sequence comprising from the N-terminus the first five transmembrane domains of a GPCR, a fusion partner domain, and the sixth and seventh transmembrane domains of such GPCR.

In certain embodiments, the invention provides a method for selecting a suitable candidate fusion partner for further evaluation for use according to the invention. In certain such embodiments, the invention provides a method including the process of screening multiple candidate fusion partners for which data is available regarding such candidate fusion partners comprising the step of selecting candidate fusion partners meeting one or more of the following criteria: (i) having N and C termini separated by no more than 15 Å, or 10 Å, or 5 Å; (ii) having a molecular weight of less than 25kD (or 20kD or 15kD); (iii) having been demonstrated to be crystallized with a diffraction resolution of at least 3 Å or 2.9 Å or 2.8 Å or 2.7 Å or 2.6 Å or 2.5 Å or 2.4 Å or 2.3 Å; (iv) having the capacity to form crystals in more than one set of chemical conditions; and (v) having the capacity to form crystals having more than one space group. In certain embodiments, the above method is practiced without the criterion labeled no. (i) above.

### BRIEF DESCRIPTION OF THE DRAWINGS

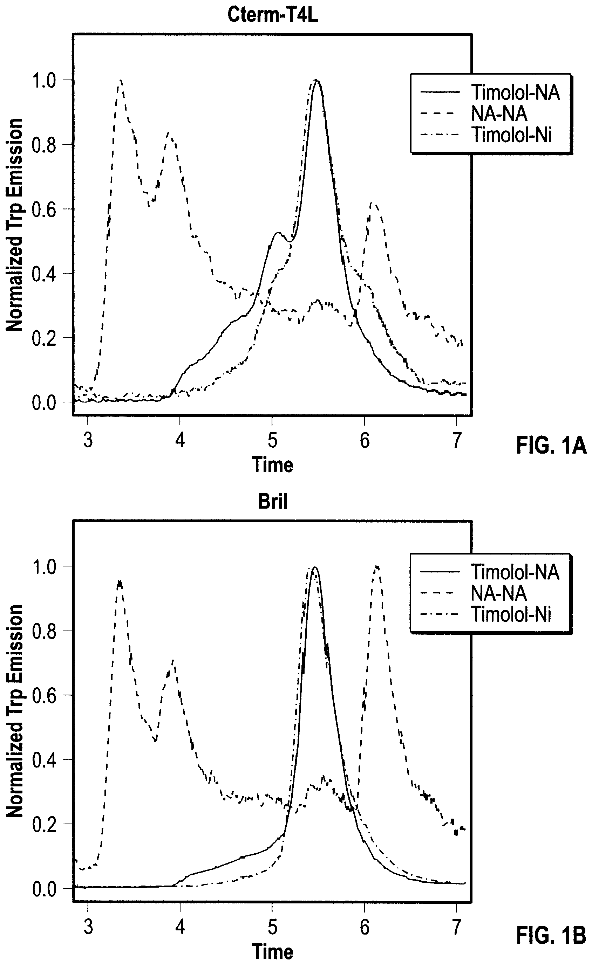

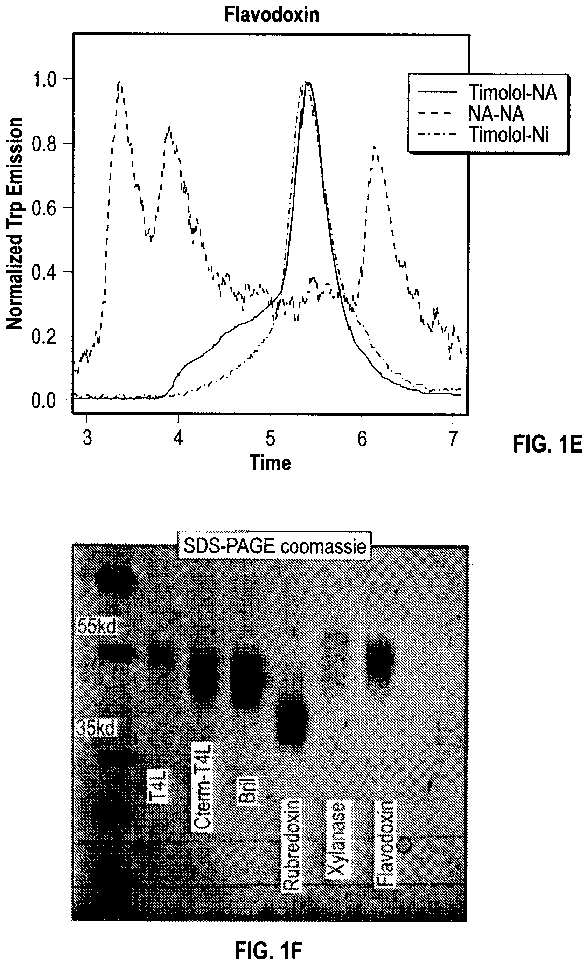

**Figures 1A-1F** show data associated with a panel of candidate fusion partners inserted into the β2-adrenergic receptor (β2AR) to form β2AR-fusion partner proteins, where Figures 1A-1E show SEC data corresponding to extraction and primary purification of each β2AR-fusion partner protein in the presence of ligand (labeled Timolol-NA) and absence of ligand (labeled NA-NA) and secondary purification of the ligand-bound species of β2AR-fusion partner proteins (labeled Timolol-Ni), and Figure 1F shows SDS-PAGE on a 10% gel for the final purified product of each β2AR-fusion partner protein, from left to right, MW standards, β2AR-T4L, β2AR-C-term-T4L, β2AR-Bril, β2AR-rubredoxin, β2AR-xylanase, and β2AR-flavodoxin.

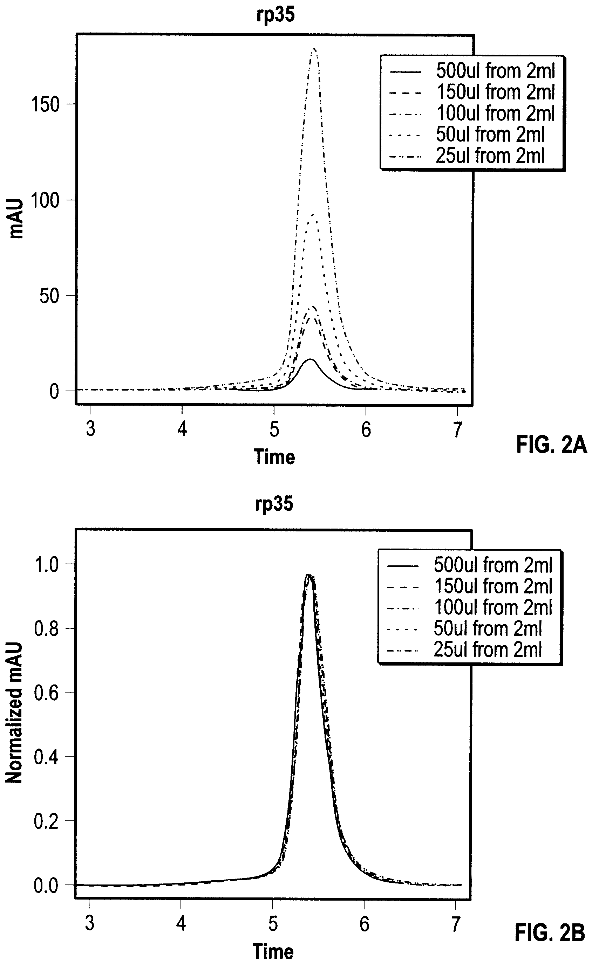

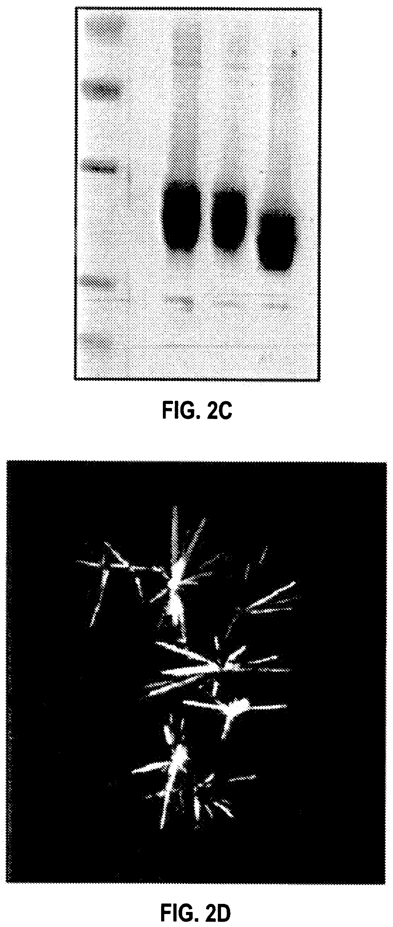

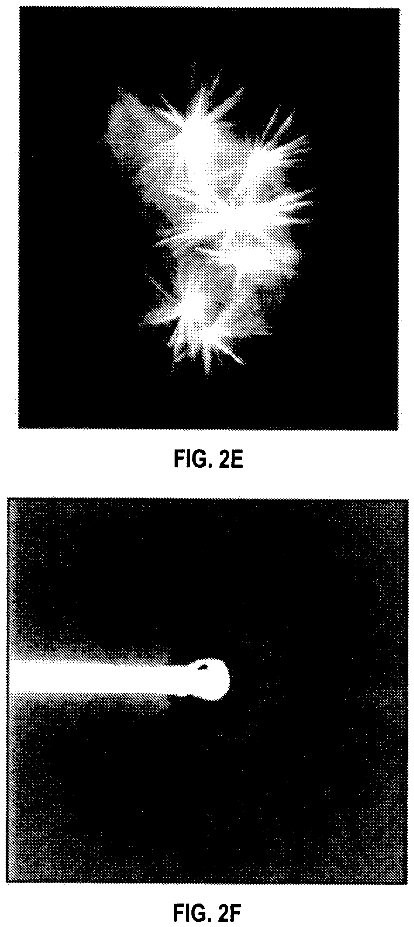

**Figures 2A-2F** show data associated with the stabilized β2AR-cytochrome B562 fusion partner protein (β2AR-Bril) crystallization and preliminary diffraction, where Figures 2A-2C show that the β2AR-Bril construct was expressed and purified (Figure 2A, analytical SEC trace of aliquots from different concentrations showing amount of protein; Figure 2B, SEC trace of aliquots from different concentrations normalized for amount of protein present), resulting in high quality monodisperse protein (Figure 2C, SDS-PAGE of β2AR-Bril after SEC), and Figures 2D-2F show that purified β2AR-Bril could form crystals, yielding large, birefringent crystals that grew large in one dimension (Figure 2D, digitized image showing birefringence of β2AR-Bril crystals) and were confirmed to be protein crystals by tryptophan fluorescence (Figure 2E, digitized image showing tryptophan fluorescence of the β2AR-Bril crystals) and diffraction (Figure 2F, digitized image of diffraction of β2AR-Bril crystals).

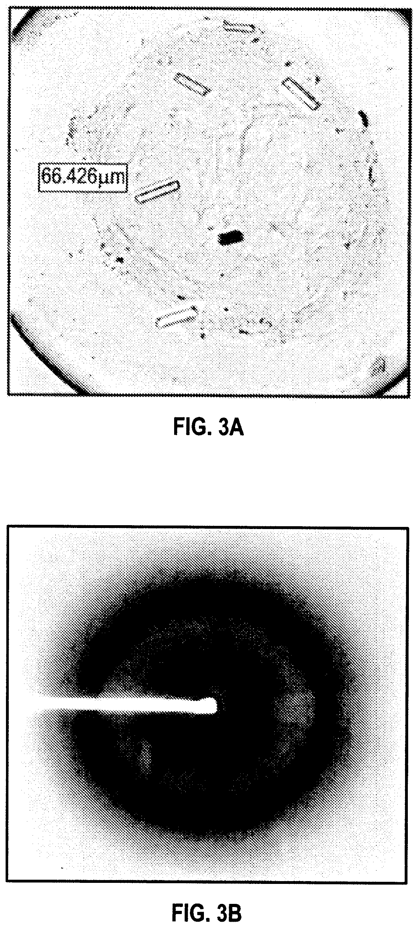

**Figures 3A-3B** show data associated with β2AR-Bril crystals resulting from inclusion of the crystallant additive tri-methyl amine N-oxide, that that results in superior crystal growth and diffraction properties, where Figure 3A shows a digitized image of β2AR-Bril crystals obtained under optimized conditions with tri-methyl amine N-oxide additive present, and Figure 3B shows diffraction results indicating these crystals diffract x-rays to a nominal resolution of 2.8 Å.

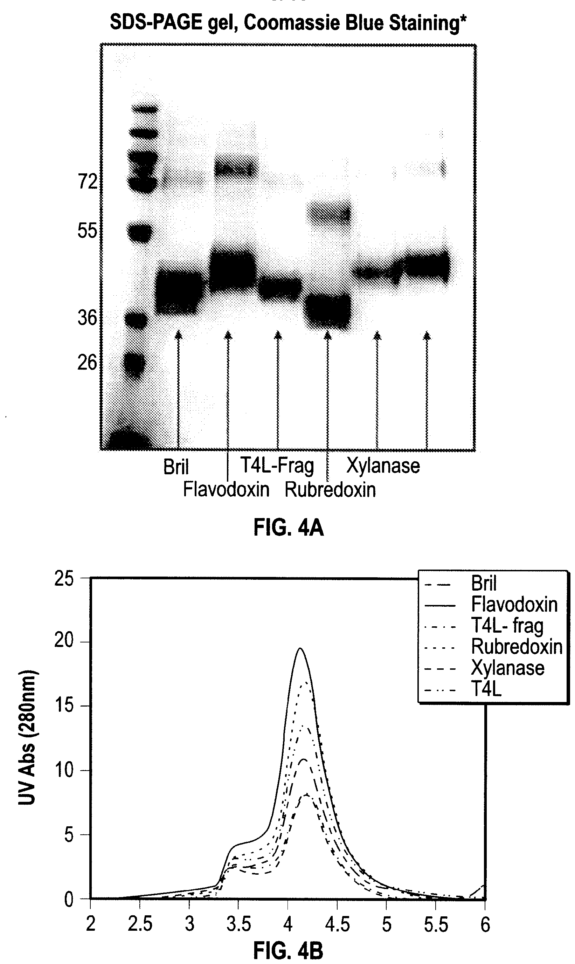

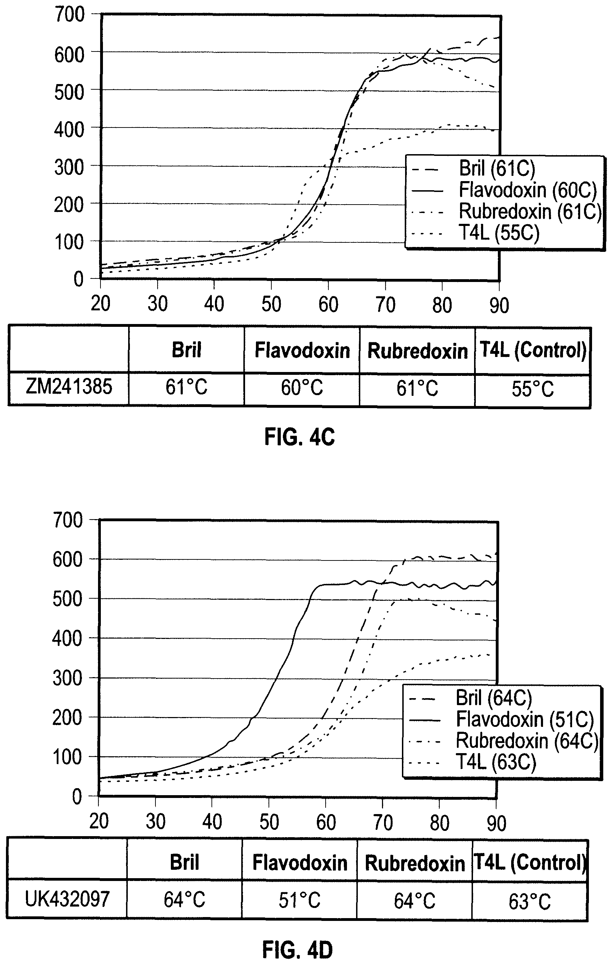

**Figures 4A-4D** show data associated with a panel of candidate fusion partners inserted into the adenosine A2A receptor (A_{2A}), where Figure 4A is a digitized image of a 10% SDS-PAGE gel characterizing expression and purification of, from left to right after MW standards in far left lane, A_{2A}-BRIL, A_{2A}-flavodoxin, A_{2A}-T4L-Cterm, A_{2A}-rubredoxin, A_{2A}-xylanase, A_{2A}-T4L, Figure 4B shows traces (protein level measured as UV absorption at 280 nm) for analytical size exclusion chromatography (aSEC) analysis of each A_{2A}-fusion partner protein for analysis of monodispersity and homogeneity, and Figures 4C and 4D show data from thermal denaturation assays measuring stability induction of known adenosine A_{2A} receptor ligands, for A_{2A}-BRIL, A_{2A}-flavodoxin, A_{2A}-rubredoxin, and A_{2A}-T4L in the presence of A_{2A} antagonist ZM241285 (Figure 4C) and in the presence of A_{2A} agonist UK432097 (Figure 4D).

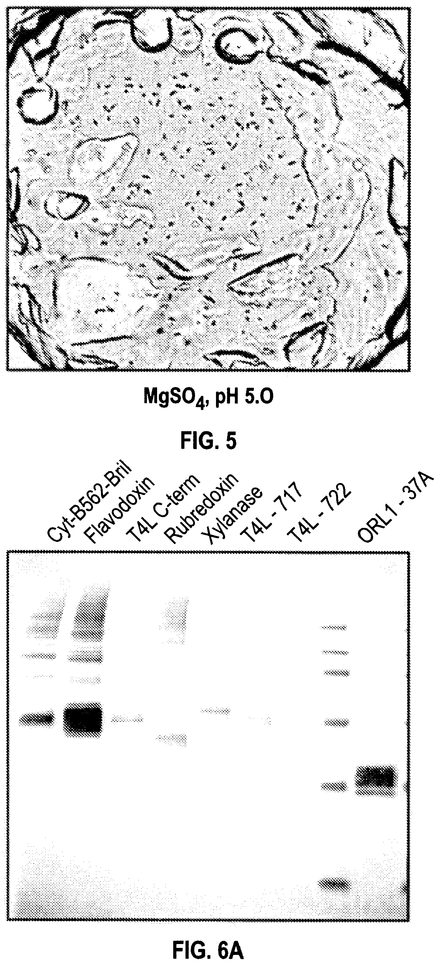

**Figure 5** shows a digitized image of crystals of A_{2A}-BRIL bound to agonist UK432097.

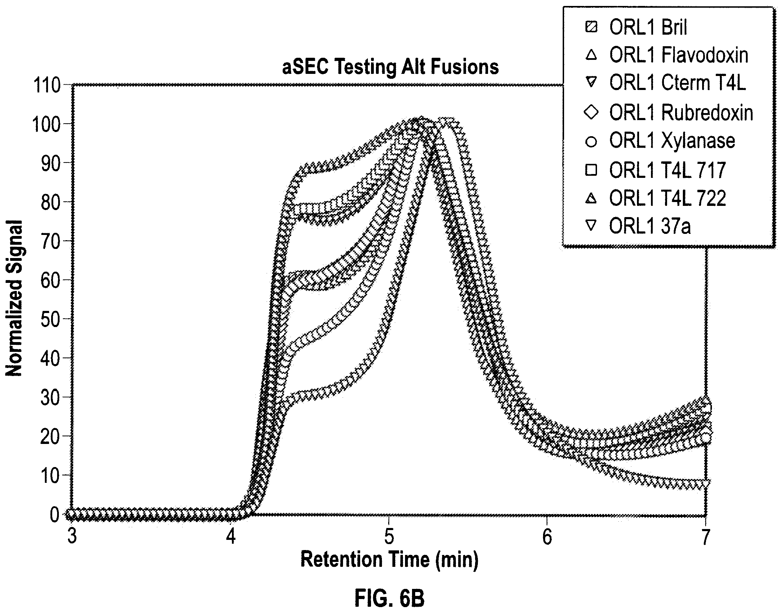

**Figures 6A-6B** shows data associated with a panel of candidate fusion partners inserted into the NOP1 receptor, where Figure 6A is a digitized image of Western blot of a 10% SDS-PAGE gel probed with anti-FLAG antibody, characterizing expression and purification of, from left to right, NOP1-BRIL, NOP1-flavodoxin, NOP1-T4L-Cterm, NOP1-rubredoxin, NOP1-xylanase, NOP1-T4L-727, NOP1-T4L-722, MW standards, NOP1-37A, and Figure 6B shows traces for analytical size exclusion chromatography (aSEC) analysis of each NOP1-fusion partner protein for analysis of monodispersity and homogeneity.

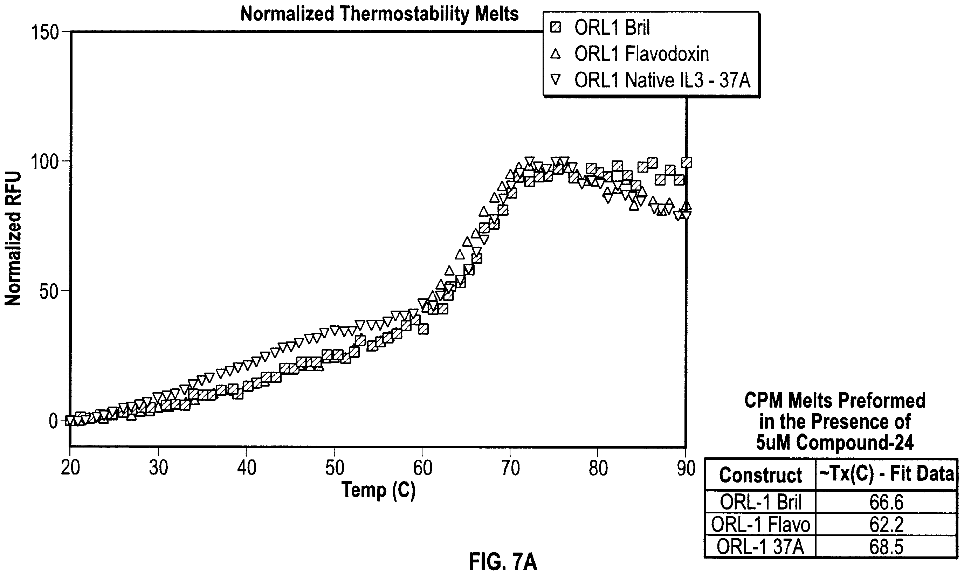

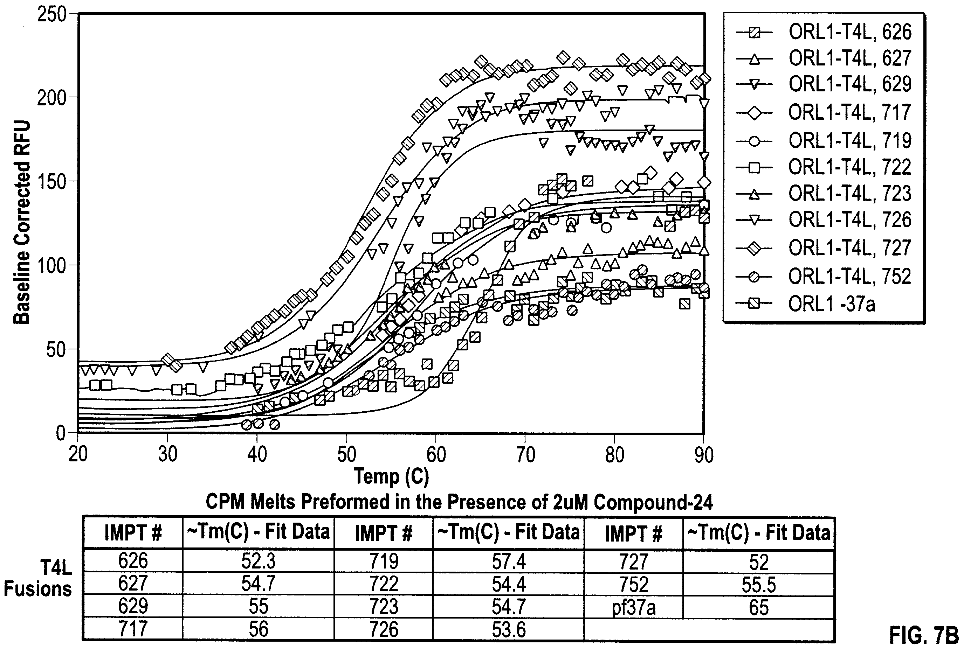

**Figures 7A-7B** show results from thermal denaturation assays comparing results for NOP1-BRIL, NOP1-flavodoxin, and NOP1 native IL3-37A (control) in Figure 7A, with results for multiple variants of NOP1-T4L (Figure 7B).

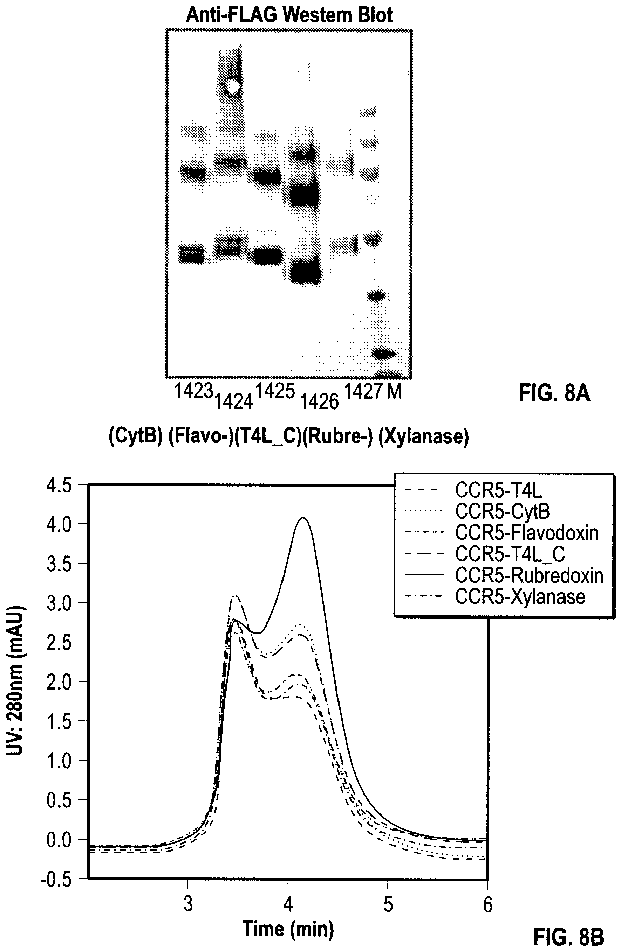

**Figures 8A-8B** show data associated with a panel of candidate fusion partners inserted into the CCR5 receptor, where Figure 8A is a digitized image of Western blot of a 10% SDS-PAGE gel probed with anti-FLAG antibody, characterizing expression and purification of, from left to right, CCR5-BRIL (labeled "1423 (CytB)"), CCR5-flavodoxin (labeled "1424 (Flavo-)"), CCR5-T4L-Cterm (labeled "1425 (T4L_C)"), CCR5-rubredoxin (labeled "1426 (Rubre-)"), CCR5-xylanase (labeled "1427 (Xylanase)"), and MW standards, and Figure 6B shows traces for analytical size exclusion chromatography (aSEC) analysis of CCR5-T4L, CCR5-BRIL ("CCR5-CytB"), CCR5-flavodoxin, CCR5-T4L-Cterm (labeled CCR5-T4L_C"), CCR5-rubredoxin, and CCR5-xylanase, for analysis of monodispersity and homogeneity.

### DETAILED DESCRIPTION OF THE INVENTION

The invention relates to a composition comprising a GPCR-fusion partner protein which comprises, from N-terminus to C terminus: (i) a first domain comprising a portion of a G-protein-coupled receptor (GPCR) wherein the first domain comprises the first through fifth transmembrane domains of the GPCR, (ii) a second domain comprising a fusion partner amino acid sequence wherein said sequence is at least 90% homologous to rubredoxin, and (iii) a third domain comprising a portion of the GPCR wherein the third domain comprises the sixth and seventh transmembrane domains of the GPCR.

The invention also concerns a composition comprises a GPCR-fusion partner protein which comprises, from N-terminus to C terminus: (i) a first domain comprising a G-protein-coupled receptor (GPCR), and (ii) a second domain comprising a fusion partner amino acid sequence wherein said sequence is at least 90% homologous to rubredoxin.

The invention further relates to a composition composition comprises a GPCR-fusion partner protein which comprises, from N-terminus to C terminus: (i) a first domain comprising a fusion partner amino acid sequence wherein said sequence is at least 90% homologous to rubredoxin, and (ii) a second domain comprising a GPCR.

In an embodiment of the invention the composition the GPCR-fusion partner protein is in crystalline form. In this embodiment the GPCR-fusion partner protein is of sufficient quality to support x-ray crystallographic structure determination for the GPCR at a resolution of at least 3 angstroms.

In another embodiment of the invention the composition the fusion partner substantially comprises the domain corresponding to the third intracellular domain of the GPCR between the fifth and sixth transmembrane domains of the GPCR.

The invention also relates to a method of using a fusion partner to support crystallization of a protein suitable for crystallographic structural studies of a G-protein-coupled receptor (GPCR) comprising: (a) incorporating a fusion partner into an intracellular domain of the GPCR to form a GPCR-fusion partner protein, wherein the fusion partner comprises an amino acid sequence which is at least 90% homologous with the amino acid sequence of rubredoxin; (b) expressing and purifying the GPCR-fusion partner protein; and (c) crystallizing the purified GPCR-fusion partner protein.

The invention further relates to a method that uses a fusion partner to support crystallization of a protein suitable for crystallographic structural studies of a G-protein-coupled receptor (GPCR) comprising: (a) attaching a fusion partner to an N-terminus or C-terminus of the GPCR to form a GPCR-fusion partner protein, wherein the fusion partner comprises an amino acid sequence which is at least 90% homologous with the amino acid sequence of rubredoxin; (b) expressing and purifying the GPCR-fusion partner protein; and (c) crystallizing the purified GPCR-fusion partner protein.

In an embodiment of the invention the method the GPCR is a class A GPCR.

In another embodiment of the invention the method further comprises the step of crystallizing the purified GPCR-fusion partner protein is performed in the presence of a crystallant additive.

Methods and compositions are provided. Compositions are provided in the form of compositions comprising fusion partner proteins combining a GPCR and a fusion partner where the fusion partner is selected according to principles set forth herein. In certain embodiments, the fusion partner is selected from rubredoxin. In certain embodiments the fusion partner protein amino acid sequence is a sequence which is at least 90% homologous to one of rubredoxin. In certain embodiments the fusion partner protein amino acid sequence is a sequence which is at least 70% homologous to one of rubredoxin and demonstrates fold similarity with the native form of such fusion partner.

GPCRs as described herein include both naturally occurring GPCRs as well as variants thereof and can include proteins having the seven transmembrane domains of a GPCR with substitutions or deletions in one or more intracellular or extracellular regions or at either or both termini. In certain embodiments the invention provides for a fusion partner protein construct comprised of a GPCR with a fusion partner substituted for some or substantially all of the intracellular domain between the fifth and sixth transmembrane domains. In certain embodiments, the invention provides for a fusion partner protein construct comprised of a GPCR or a GPCR truncated at the C-terminus and/or the N-terminus with a fusion partner attached at either the C-terminus or the N-terminus of such GPCR or truncated GPCR.

Methods are provided for using new fusion partners to support crystallization of a protein suitable for crystallographic structural studies of a GPCR, *e.g.,* for structure determination of the GPCR by X-ray crystallography of the GPCR-fusion partner protein. Compositions resulting from the present methods are provided.

Methods are provided for using new fusion partners to support crystallization of a protein suitable for crystallographic structural studies of a GPCR by incorporating a fusion partner into an intracellular domain of the GPCR to form a GPCR-fusion partner protein, wherein the fusion partner includes an amino acid sequence of rubredoxin expressing and purifying the GPCR-fusion partner protein, followed by crystallizing the purified GPCR-fusion partner protein, conducting crystallographic structural studies of the crystallized GPCR-fusion partner protein, and determining structural features of the GPCR from the crystallographic structural studies of the crystallized GPCR-fusion partner protein.

Methods are provided for using new fusion partners to support crystallization of a protein suitable for crystallographic structural studies of a GPCR by attaching a fusion partner to the N-terminus or the C-terminus of the GPCR to form a GPCR-fusion partner protein, wherein the fusion partner includes an amino acid sequence of rubredoxin expressing and purifying the GPCR-fusion partner protein, followed by crystallizing the purified GPCR-fusion partner protein, conducting crystallographic structural studies of the crystallized GPCR-fusion partner protein; and determining structural features of the GPCR from the crystallographic structural studies of the crystallized GPCR-fusion partner protein.

Methods are provided for using new fusion partners to improve crystallization of GPCR-fusion partner proteins to yield GPCR-fusion partner proteins suitable for crystallographic structural studies, to support. Methods are provided for using new fusion partners to improve expression of GPCR-fusion partner proteins, to support crystallization of GPCR-fusion partner proteins. Methods are provided for using new fusion partners to improve purification of GPCR-fusion partner proteins, to support crystallization of GPCR-fusion partner proteins.

Methods are provided for identifying, predicting, selecting, screening, and evaluating potential new fusion partners for incorporation into an available intracellular loop of a GPCR to yield a GPCR-fusion partner protein having desired biochemical and structural properties for crystallization and structure determination by X-ray crystallography. Methods are provided for identifying, selecting, screening, evaluating GPCR-fusion partner proteins.

Methods are provided for searching, screening, and mining databases to identify new protein structures, domain structures, proteins, polypeptides, domains, or their equivalents, that are capable of being used as fusion partners to support crystallization of GPCR-fusion partner proteins suitable for crystallographic structural studies.

Methods are provided for identifying, predicting selecting, screening, and evaluating potential new fusion partners that are suitable as fusion partners to support crystallization of GPCR-fusion partner proteins suitable for crystallographic structural studies. In certain such embodiments the fusion partners provide improved performance as compared with T4L.

Methods are provided for searching, screening, and mining databases to identify new protein structures, domain structures, proteins, polypeptides, domains, or their equivalents, that are capable of providing suitable fusion partners to support crystallization of GPCR-fusion partner proteins suitable for crystallographic structural studies. In certain such embodiments the fusion partners provide improved performance as compared with T4L.

GPCR-fusion partner proteins having desired biochemical and structural properties for crystallization and crystallographic structural studies are provided.

Crystals of GPCR-fusion partner proteins having desired biochemical and structural properties for crystallization and crystallographic structural studies are provided.

Methods are provided for identifying potential new fusion partners for incorporation into an available intracellular loop of a GPCR to yield a GPCR-fusion partner protein having desired biochemical and structural properties for crystallization and structure determination by X-ray crystallography. GPCR-fusion partner proteins according to the present methods for identifying are provided.

Methods are provided for selecting potential new fusion partners for incorporation into an available intracellular loop of a GPCR, or attachment to an N-terminus or a C-terminus of a GPCR, to yield a GPCR-fusion partner protein having desired biochemical and structural properties for crystallization and structure determination by X-ray crystallography. GPCR-fusion partner proteins according to the present methods for selecting are provided.

Methods are provided for screening potential new fusion partners for incorporation into an available intracellular loop of a GPCR, or attachment to an N-terminus or a C-terminus of a GPCR, to yield a GPCR-fusion partner protein having desired biochemical and structural properties for crystallization and structure determination by X-ray crystallography. GPCR-fusion partner proteins according to the present methods for screening are provided.

Methods are provided for evaluating potential new fusion partners for incorporation into an available intracellular loop of a GPCR, or attachment to an N-terminus or a C-terminus of a GPCR, to yield a fusion partner protein having desired biochemical and structural properties for crystallization and structure determination by X-ray crystallography. GPCR-fusion partner proteins according to the present methods for evaluating candidate fusion partners are provided.

Methods are described for using new fusion partners including *E. coli* flavodoxin (PDB ID:1AG9, MW: 20 kD), *M. tuberculosis* hypothetical protein, (PDB ID: 2ASF, MW: 15 kD), *E coli* CHEY (PDB ID: 1JBE, MW: 14kD), *Bos taurus* BPT1 (PDB ID: 1G6X, MW: 7 kD), T4 lysozyme C-terminal fragment ("Cterm-T4L" PDB ID: 2O7A, T4 Enterobacteria phage, 14 kD); flavodoxin (PDB ID: 1I1O, *Desulfovibrio vulgaris,* mutant Y98H, 16kD); Cytochrome b₅₆₂ RIL ("Bril" PDB ID: 1M6T, *Escherichia coli* soluble cytochrome b562, 12 kD); and chemotaxis protein cheA (PDB ID: 1TQG, CheA phosphotransferase domain from *Thermotoga maritima*, 11.9 kD). In certain embodiments, the invention provides fusion partners which have sequence homology of 70%, 75%, 80%, 85%, 90%, 95%, 98%, or 99% sequence homology with any of the above fusion partners and in certain embodiments also having a high degree of fold similarity or substantial fold similarity with such fusion partner.

Methods are providing for using new fusion partners to support crystallization of a protein suitable for crystallographic structural studies of GPCRs including 5-HT1A, 5-HT1B, 5-HT1D, 5-htle, 5-HT1F, 5-HT2A, 5-HT2B, 5-HT2C, 5-HT4, 5-ht5a, 5-HT6, 5-HT7, M1, M2, M3, M4, M5, A1, A2A, A2B, A3, alpha 1A-adrenoceptor, alpha 1B-adrenoceptor, alpha 1D-adrenoceptor, alpha 2A-adrenoceptor, alpha 2B-adrenoceptor, alpha 2C-adrenoceptor, beta 1-adrenoceptor, beta 2-adrenoceptor, beta 3-adrenoceptor, C3a, C5a, C5L2, AT1, AT2, APJ, GPBA, BB1, BB2, BB3, B1, B2, CB1, CB2, CCR1, CCR2, CCR3, CCR4, CCR5, CCR6, CCR7, CCR8, CCR9, CCR10, CXCR1, CXCR2, CXCR3, CXCR4, CXCR5, CXCR6, CXCR7, CX3CR1, XCR1, CCK1, CCK2, D1, D2, D3, D4, D5, ETA, ETB, GPER, FPR1, FPR2/ALX, FPR3, FFA1, FFA2, FFA3, GPR42, GAL1, GAL2, GAL3, ghrelin, FSH, LH, TSH, GnRH, GnRH2, H1, H2, H3, H4, HCA1, HCA2, HCA3, kisspeptin, BLT1, BLT2, CysLT1, CysLT2, OXE, FPR2/ALX, LPA1, LPA2, LPA3, LPA4, LPA5, S1P1, S1P2, S1P3, S1P4, S1P5, MCH1, MCH2, MC1, MC2, MC3, MC4, MC5, MT1, MT2, motilin, NMU1, NMU2, NPFF1, NPFF2, NPS, NPBW1, NPBW2, Y1, Y2, Y4, Y5, NTS1, NTS2, delta , kappa , mu , NOP, OX1, OX2, P2Y1, P2Y2, P2Y4, P2Y6, P2Y11, P2Y12, P2Y13, P2Y14, QRFP, PAF, PKR1, PKR2, PRRP, DP1, DP2, EP1, EP2, EP3, EP4, FP, IP1, TP, PAR1, PAR2, PAR3, PAR4, RXFP1, RXFP2, RXFP3, RXFP4, sst1, sst2, sst3, sst4, sst5, NK1, NK2, NK3, TRH1, TA1, UT, VIA, V1B, V2, OT, CCRL2, CMKLR1, GPR1, GPR3, GPR4, GPR6, GPR12, GPR15, GPR17, GPR18, GPR19, GPR20, GPR21, GPR22, GPR25, GPR26, GPR27, GPR31, GPR32, GPR33, GPR34, GPR35, GPR37, GPR37L1, GPR39, GPR42, GPR45, GPR50, GPR52, GPR55, GPR61, GPR62, GPR63, GPR65, GPR68, GPR75, GPR78, GPR79, GPR82, GPR83, GPR84, GPR85, GPR87, GPR88, GPR101, GPR119, GPR120, GPR132, GPR135, GPR139, GPR141, GPR142, GPR146, GPR148, GPR149, GPR150, GPR151, GPR152, GPR153, GPR160, GPR161, GPR162, GPR171, GPR173, GPR174, GPR176, GPR182, GPR183, LGR4, LGR5, LGR6, LPAR6, MAS1, MAS1L, MRGPRD, MRGPRE, MRGPRF, MRGPRG, MRGPRX1, MRGPRX2, MRGPRX3, MRGPRX4, OPN3, OPN5, OXGR1, P2RY8, P2RY10, SUCNR1, TAAR2, TAAR3, TAAR4 , TAAR5, TAAR6, TAAR8, TAAR9, CCPB2, CCRL1, FY, CT, calcitonin receptor-like, CRF1, CRF2, GHRH, GIP, GLP-1, GLP-2, glucagon, secretin, PTH1, PTH2, PAC1, VPAC1, VPAC2, BAI1, BAI2, BAI3, CD97, CELSR1, CELSR2, CELSR3, ELTD1, EMR1, EMR2, EMR3, EMR4P, GPR56, GPR64, GPR97, GPR98, GPR110, GPR111, GPR112, GPR113, GPR114, GPR115, GPR116, GPR123, GPR124, GPR125, GPR126, GPR128, GPR133, GPR143, GPR144, GPR157, LPHN1, LPHN2, LPHN3, CaS, GPRC6, GABAB1, GABAB2, mGlu1, mGlu2, mGlu3, mGlu4, mGlu5, mGlu6, mGlu7, mGlu8, GPR156, GPR158, GPR179, GPRC5A, GPRC5B, GPRC5C, GPRC5D, frizzled, FZD1, FZD2, FZD3, FZD4, FZD5, FZD6, FZD7, FZD8, FZD9, FZD10, SMO. GPCRs to be evaluated include: Class A Rhodopsin-like GPCRs; Class B Secretin-like GPCRs; Class C Metabotropic glutamate/pheromone GPCRs; cAMP receptors Vomeronasal receptors (V1R and V3R); and Taste receptors T2R. GPCRs to be evaluated include, but are not limited to, a class A GPCR, a class B GPCR, a class C GPCR, a class D GPCR, a class E GPCR, and a class F GPCR.

Candidate GPCRs include β2-adrenergic receptor (β2AR), A2A-Adenosine Receptor (A_{2A}), S1P1, Opioid receptor (OLR) including NOP 1, Chemokine receptor CXCR3 (CXCR3), Chemokine receptor CCR5 (CCR5), GLP1R, PTHR1, LPA1, LPA2, LPA3, S1P2, S1P3, S1P4, and S1P5.

### Evaluation Criteria and Parameters

Methods are provided herein to utilize at least some of the following criteria to search, screen, or "mine" databases of protein data in order to identify and evaluate potential fusion partners and the resulting GPCR-fusion partner protein. In accordance with one aspect, methods are provide that utilize at least the following criteria to identify and evaluate potential fusion partners to provide suitable fusion partners to support crystallization of GPCR-fusion partner proteins suitable for crystallographic structural studies, including in certain embodiments fusion partners which provide improved performance as compared with T4L. Initial search criteria include to criteria in Table 1 below:

| **Table 1 Non-Limiting List of Initial Search Criteria** | |
|---|---|
| **Search parameter** | **Criterion for potential fusion partner** |
| Relative location of N and C termini | and C termini of potential fusion partner should be separated by no more than 15 Å |
| Molecular weight of purified protein | |
| | Less than 25 kD |
| Crystal structure of purified protein | Crystal of potential fusion partner should have diffraction resolution better than 3 Å |
| Requirements for crystal formation | |
| | Crystal of potential fusion partner should form |
| | in at least two different chemical conditions |
| Crystal packing of purified protein | Crystal of potential fusion partner should result in more than one space group |
| | |

Additional features or criteria for selecting and evaluating potential new fusion partner can include relative features, *i.e.,* a set of potential new fusion partners may be selected having different parameter values such as size (kD), number of folds (fold diversity).

In certain embodiments where fusion partners are sought for evaluation for use as a fusion partner attached to a terminus of a GPCR, the criterion regarding the proximity of the fusion partners N and C termini may be given less weight or no weight.

In certain embodiments, the invention provides a method for selecting a suitable candidate fusion partner for further evaluation for use according to the invention. In certain such embodiments, the invention provides a method including the process of screening multiple candidate fusion partners for which data is available regarding such candidate fusion partners comprising the step of selecting candidate fusion partners meeting one or more of the following criteria: (i) having N and C termini separated by no more than 15 Å, or 10 Å, or 5 Å; (ii) having a molecular weight of less than 25kD (or 20kD or 15kD); (iii) having been demonstrated to be crystallized with a diffraction resolution of at least 3 Å or 2.9 Å or 2.8 Å or 2.7 Å or 2.6 Å or 2.5 Å or 2.4 Å or 2.3 Å; (iv) having the capacity to form crystals in more than one set of chemical conditions; and (v) having the capacity to form crystals having more than one space group. In certain embodiments, the above method is practiced without the criterion labeled no. (i) above. In certain embodiments, the above method is practiced by also applying the following additional criteria: (vi) having at least 50% alpha-helical content; and (vii) having alpha-helical domains at the N-terminus, or the C-terminus or both. Without being bound by any particular theory it is proposed that high alpha-helical content correlates with faster folding kinetics which may improve stability and correct folding of the fusion protein. Additionally, alpha-helical domains at the termini may promote more stable folding by continuing the alpha-helical motif of an adjacent transmembrane domain of the GPCR, particularly so if the junction is chosen for continuity of the alpha-helical domains of the GPCR and the fusion partner such that a continuous alpha-helical domain spans a junction (or both junctions) between the GPCR and the fusion partner.

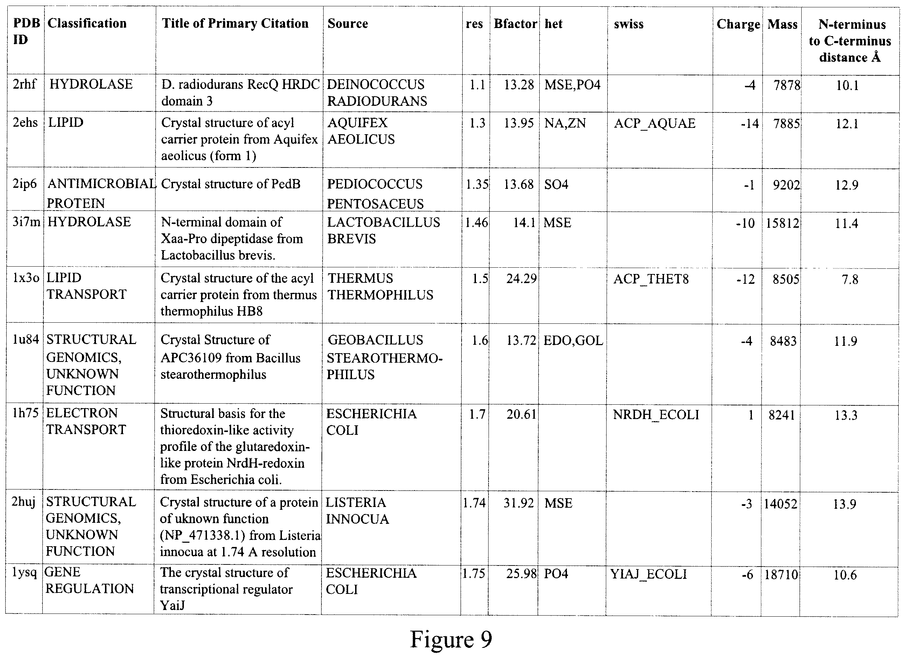

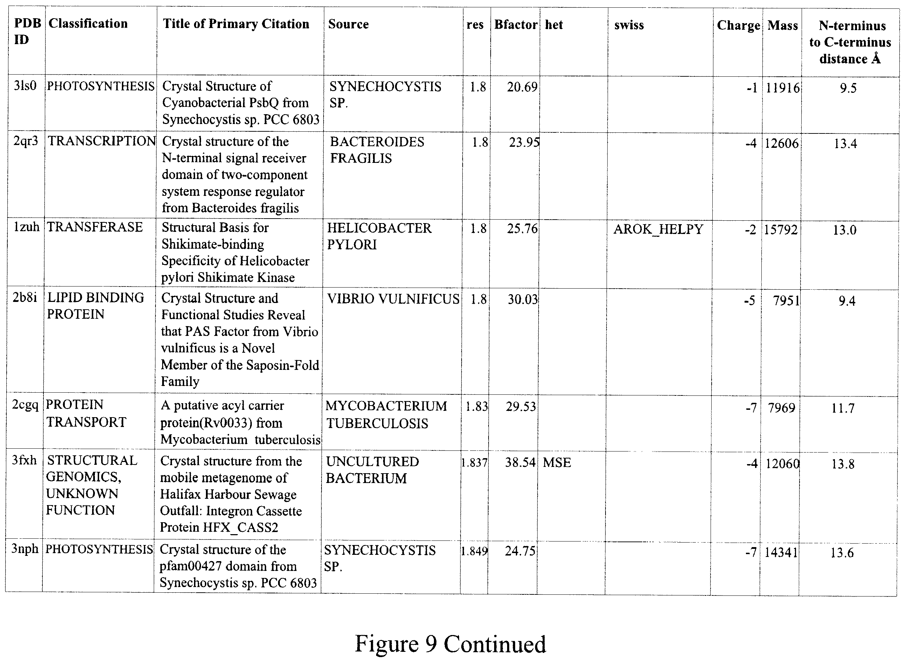

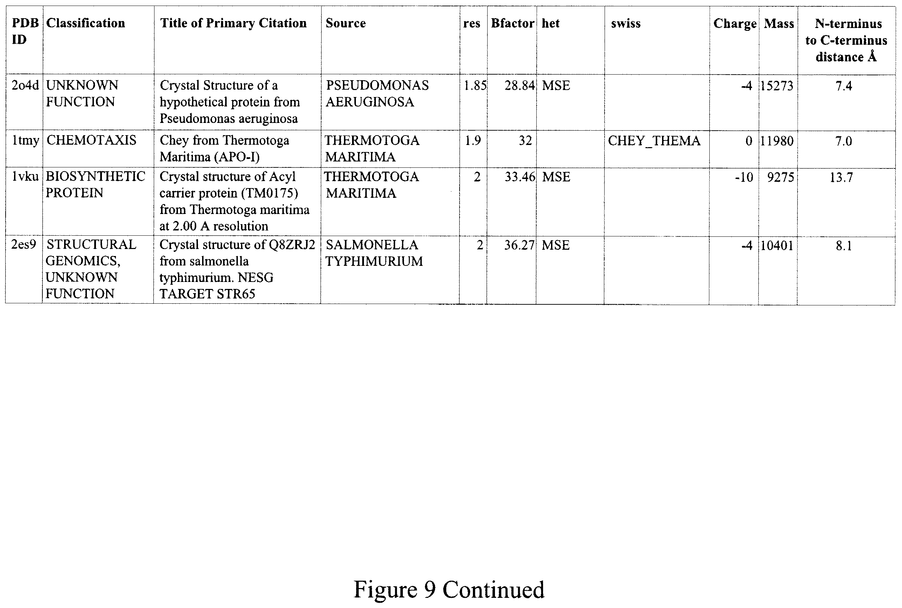

By applying the methods of the invention including the criteria (i) - (vii), the following candidate fusion partners were identified (listed using Protein Data Bank IDs): 2rhf, 2ehs, 2ip6, 3i7m, lx3o, 1u84, 1h75, 2huj, lysq, 31s0, 2qr3, 1zuh, 2b8i, 2cgq, 3fxh, 3nph, 2o4d, 1tmy, 1vku, and 2es9. Summary results of such search and identified candidate fusion partners meeting the criteria (i) -(vii) are shown in Figure 9.

### Searching, screening, mining, identifying potential fusion partners

Methods are provided herein to search, screen, and/or "mine" searchable databases of protein information for database entries for potential fusion partners that satisfy at least the criteria listed in Table 1. Methods are provided herein for identifying potential fusion partners that satisfy at least the criteria used in the searching, screening, and/or mining methods provided herein. Methods are provided herein for identifying potential fusion partners that satisfy at least the criteria used in the searching, screening, and/or mining methods provided herein, to provide suitable fusion partners to support crystallization of GPCR-fusion partner proteins suitable for crystallographic structural studies. In cettain embodiments, fusion partners are provided which have improved performance as compared with T4L.

Exemplary databases include the Protein Data Bank (PDB; Worldwide Protein Data Bank, www.wwpdb.org), JenaLib; ModBase; OCA; SCOP; CATH; Iditis.

One could further characterize potential fusion proteins by parameters including their experimental flexibility, experimental thermal stability, preselect structures from thermophiles and exclude proteins with co-factor or metal binding requirements.

Methods are provided herein to further evaluate potential fusion partners identified by database searching, screening, and/or mining methods provided herein. Additional criteria are used to evaluate the information provided in one or more databases for each potential fusion partner identified using the initial search criteria of Table 1.

Methods are provided for using an object-oriented structured approach to screening the collection of molecular structures archived in a database (entries contained in a database). In accordance with one aspect, an object-oriented structured approach is provided to screen the molecular structures ("objects") archived in a database in a compatible format, and operations are performed on the "objects" including exploration of relevant metadata such as experimental conditions, diffraction resolution, and authorship, permitting the user to programmatically filter and/or screen database entries based on any variety of metadata the user specifies within the graphical user interface (GUI). In accordance with another aspect, a GUI is provided with interface controls that enable the user to explore some, many, or all attributes provided by the database.

In an embodiment, Visual Studio Professional 2008 (Microsoft Corporation) is used to develop applications to carrying out the presently provided methods, leveraging the .NET framework v.3.5 (Microsoft Corporation) including the Windows Presentation Foundation (WPF) graphical subsystem of and the C# programming language ("C Sharp Language" or "C# Language"). In a further non-limiting embodiment, the Windows Presentation Foundation (WPF) is a graphical subsystem of the .NET framework v.3.5 providing a programming model for building applications, that allows for separation between the business logic of the application such as programmatic filtering and/or backend processing, and the graphical using interface (GUI) of the software client application.

In an embodiment, an object-oriented structured approach is used to screen the molecular structures archived in the PDB repository, in an PDBXML file for each protein structure, and each PDBXML file representing a single protein structure (an "object") is referenced within a .Net data structure compatible with the XML format, and operations are performed on the "object" including exploration of relevant metadata such as experimental conditions, diffraction resolution, and authorship, permitting the user to programmatically filter and/or screen database entries based on any variety of metadata the user specifies within the graphical user interface (GUI). In another non-limiting embodiment, the GUI includes strategic interface controls that enable the user to explore all attributes provided by the database, *e*.*g*., the RCSB repository.

Methods are provided for mining databases for potential fusion partners, where mining can occur once or multiple times, and mining can be continuous or intermittent. Methods for mining databases are provided for retrieval of entries contained within a database, to collect data in order to explore available structures. Methods are provided to synchronize local data storage with the database. In accordance with one aspect, methods are provided for automated retrieval of entries contained within a database, including automated retrieval of all entries contained within a database, automated retrieval of some entries contained within a database, automated retrieval of selected entries contained within a database, and automated retrieval of entries that satisfy one or more search criteria. Methods are further provided for retrieval of updates including but not limited to recent deposits (new entries) in the database, corrections of entries contained in the database, and further annotations of entries contained in the database, where updates can be retrieved automatically, or by other means such as scheduled searches or retrieval. In accordance with one aspect, automated data collection includes a full download of all existing entries in a database, and local incremental automatic update of recent deposits and other updates to the database.

In an embodiment, data is collected by automated retrieval of all entries in a database. In another embodiment, data is collected by an initial automated retrieval of all entries in a database, followed by local incremental update of recent deposits to the database. In a non-limiting example, automated retrieval of all Protein Data Back (PDB) entries contained within the Research Collaboratory for Structural Bioinformatics (RCSB) consisted of (1) full download of all existing PDB entries wherein the local data store was synchronized with the PDB's PDBXML repository, and (2) local incremental update of recent deposits to the RCSB repository. In this example, data was synchronized locally using the http: protocol to obtain the files directly from the wwPDB's worldwide collection of servers (via www.wwpdb.org). In various non-limiting embodiments, the collection all Protein Data Bank (PDB) entries ranged in size from 200 GB to 320 GB, depending on the time when the screening took place. When the PDB repository contained entries for over 60,000 structures, a local copy of the PDB's repository reached 320 GB in size.

In an embodiment, data is collected by using search criteria to identify candidates within the database, and retrieval of all entries from the database that have been identified using the search criteria.

### Evaluating new fusion partners and GPCR-fusion partner proteins

A potential new fusion partner (or, candidate fusion partner) is incorporated into at least one GPCR, in at least one location in the GPCR, and the resulting GPCR-fusion partner protein will be determined. In accordance with one aspect, each potential new fusion partner will be incorporated into at least two different receptors. In accordance with another aspect, each potential new fusion partner will be incorporated into at least two distinct locations in the same receptor, to produce at least two distinct GPCRs having the same receptor and the same fusion partner, but wherein each distinct GPCR has the fusion partner incorporated in a different location from the other. In accordance with another aspect, at least two distinct GPCR-fusion partner proteins will be made for each receptor, wherein each distinct GPCR-fusion partner protein has a different receptor.

An example of a set of potential fusion partners described herein mined from the PDB for incorporation into GPCRs to produce GPCR-fusion partner proteins includes: *E. coli* flavodoxin (PDB ID:1AG9, MW: 20 kD), *M. tuberculosis* hypothetical protein Rv2074, (PDB ID: 2ASF, MW: 15 kD), *E coli* chemotaxis protein CHEY (PDB ID: 1JBE, MW: 14kD), *Bos taurus* bovine pancreatic trypsin inhibitor BPT1 (PDB ID: 1G6X, MW: 7 kD). Another of a set of potential fusion partners described herein mined from the PDB for incorporation into GPCRs to produce GPCR-fusion partner proteins includes: T4 lysozyme C-terminal fragment (PDB ID: 2O7A, T4 Enterobacteria phage, 14 kD); flavodoxin (PDB ID: 111O, *Desulfovibrio vulgaris*, mutant Y98H, 16kD); Cytochrome b₅₆₂ RIL ("Bril" PDB ID: 1M6T, *Escherichia coli* soluble cytochrome b562, 12 kD); and chemotaxis protein cheA (PDB ID: 1TQG, CheA phosphotransferase domain from *Thermotoga maritime*, 11.9 kD). Yet another example of a new fusion partners identified by using the criteria above, having variation in size and fold (fold diversity) include Rubredoxin (PDB ID: 1FHM, *Clostridium pasteurianum* rubredoxin, 6 kD). Other examples described herein are Cytochrome b₅₆₂ RIL ("Bril" PDB ID: 1M6T, soluble cytochrome b₅₆₂, 12 kD), T4 Lysozyme C-terminal fragment (C-term-T4L" PDB ID: 2O7A, T4 Enterobacteria phage, T4 lysozyme circular permutant, 14 kD), Flavodoxin (PDB ID: 1I1O, *Desulfovibrio vulgaris,* mutant Y98H, 16 kD), and Xylanase (PDB ID: 2B45, Endo-1,4-beta-xylanase A, 21 kD) In another example described herein, a set further includes T4 Lysozyme (T4L, 18 kD) as a control.

Each GPCR-fusion partner protein is tested for crystallizability after a combinatorial insertion into the GPCR in different locations relative to the intracellular ends of TM-V and TM-VI.

GPCRs to be evaluated include a class A GPCR, a class B GPCR, a class C GPCR, a class D GPCR, a class E GPCR, and a class F GPCR, Candidate GPCRs include β2-adrenergic receptor (β2AR), A2A-Adenosine Receptor (A_{2A}), Sphingosine 1-phosphate receptor type 1 (S1P1), Opioid receptor (OLR) including NOP1, Chemokine receptor CXCR3 (CXCR3), Chemokine receptor CCR5 (CCR5).

### Incorporation of fusion partner into intracellular domain of GPCR

The optimal location for prospective fusion partners can be established through analysis of the structures solved to date. The incorporation of the fusion protein should not disrupt any secondary structure elements or tertiary packing interactions and should not interfere with the ligand binding pocket. Given these considerations for class A receptors a reliable option for loop replacement is the third intracellular loop where the structural disorder appears to be the highest. Other locations could be appropriate for other classes of 7TM receptors such as the class B GPCRs where little is known about the relative order of the regions and loops. Modifications to the precise location within the third intracellular loop are part of the process of optimizing each construct.

In certain embodiments, each GPCR-fusion partner protein is tested for crystalizability after a combinatorial insertion of the fusion partner into the GPCR in different locations relative to the intracellular ends of TM-V and TM-VI. The amount of variation will be related to the length of the loop and the sequence homology to known structures. On average, 2-3 initial insertion points are evaluated, although the invention encompasses insertion at 1, 4, 5, 5, 6, 7, 8, 9, 10, 11, 12, 13, 14, 15, or more locations relative to the intracellular ends of TM-V and TM-VI. In certain embodiments, each GPCR-fusion partner protein is tested for crystalizability after an attachment of the fusion partner to the N or C terminus of the GPCR or an N or C terminus truncated form of the GPCR.

In certain embodiments of the invention, alternative junction locations for the fusion partner between TM-V and TM-VI are evaluated for their effect on the thermal stability of fusion protein as an indicator of potential suitability for crystallization.

### Evaluation

The viability of a potential fusion partner (also referred to as, *inter alia,* fusion partner candidate protein, alternate fusion partners, potential carrier proteins, prospective fusion partner, *usw.*) identified by searching as provided herein, is evaluated as provided herein. In an exemplary embodiment, β2-adrenergic receptor (β2AR) serves as a model system in which each of six potential fusion partner proteins identified by searching as provided herein, has been cloned into the same β2AR sites as T4-lysozyme was cloned into for the β2AR-T4L from which structures were solved. That is, expression vectors for each β2AR-potential fusion partner construct were constructed to give the desired β2AR-potential fusion partner construct as an expression product, and each β2AR-potential fusion partner construct was progressed through viral generation of expression vectors, medium scale expression, and preliminary characterization of the stability and monodispersity of β2AR-potential fusion partner constructs. In an exemplary embodiment using β2AR-potential fusion partner constructs, two (2) potential fusion partner proteins were eliminated from consideration, the remaining four (4) β2AR-potential fusion partner constructs were then scaled up and the β2AR-potential fusion partner protein progressed into crystallization trials. Preliminary crystal hits were obtained for two of the four constructs that were scaled up and progressed into crystallization trials, with the β2AR-BRIL construct crystallizing in three distinct crystallization conditions. Further optimization lead to the identification of conditions sufficient to grow crystals large enough for preliminary diffraction studies to determine their potential for supporting high-resolution structural determination. In this exemplary embodiment, the best crystals diffracted to approximately 2.8 Å. In this exemplary embodiment, conditions that yielded the best crystals have been further optimized to increase size, thickness and birefringence, by adjusting parameters known to be related to attaining desired levels resolution in past crystallization efforts.

In addition to the exemplary embodiment of incorporating a variety of potential fusion partners (alternate fusion partners) into β2AR as described herein, further non-limiting embodiments have been practiced to explore the utility of a variety of potential fusion partners in multiple additional GPCRs. Each of five different potential fusion partners described herein were cloned into each of the GPCRs, adenosine A2A, NOP1, and CXCR3, with the potential fusion partner incorporated into optimized junction sites. In these further embodiments, each GPCR-potential fusion partner construct has been taken through small scale expression studies and biophysical characterizations. In certain embodiments, evaluation of adenosine A_{2A}-fusion partner proteins has further progressed into crystallization trials with preliminary results. Without wishing to be limited by this observation, overall the results are favorable, with at least two potential fusion partners yielding similar or better results when compared to T4L as a fusion partner for each target GPCR. In accordance with the methods and compositions as described herein, specific conditions for formation of the β2AR-T4L crystals generally translated to the β2AR-BRIL construct.

Methods and compositions as provided herein are practiced according to knowledge of one of skill in the art, using known methods unless otherwise indicated, in particular according to methods for assembling constructs, cloning, expression, purification, crystallization including but not limited to lipidic cubic crystallization, evaluation including but not limited to thermal stability assays, ligand binding assays including determination of saturation isotherms and competition binding assays, as disclosed *inter alia,* in Cherezov *et al.*, 2004 (Cherezov, V., Peddi, A., Muthusubramaniam, L., Zheng, Y. F., and Caffrey, M., "A robotic system for crystallizing membrane and soluble proteins in lipidic mesophases," Acta Crystallogr D Biol Crystallogr 60: 1795-1807, 2004), Cherezov et al. 2007 (Science 318: 1258-1265, 2007), Rosenbaum et al., 2007 (Science 318: 1266-1273, 2007), Stevens *et al.*, as disclosed in PCT/US08/80847 published as WO 2009/055509 A2, and in US Patent Application No. 12/739,134, published as US 20110031438 A1 (Feb. 10, 2011), T.E. Creighton, Proteins: Structures and Molecular Properties (W.H. Freeman and Company, 1993); Sambrook, et al., Molecular Cloning: A Laboratory Manual (2nd Edition, 1989); Methods In Enzymology (S. Colowick and N. Kaplan eds., Academic Press, Inc.); Remington's Pharmaceutical Sciences (Easton, Pennsylvania: Mack Publishing Company, 18th Edition, 1990); Carey and Sundberg, Advanced Organic Chemistry, Vols. A and B (Plenum Press, 3rd Edition 1992), the disclosures of which are hereby incorporated by reference in their entirety.

### EXAMPLES

### Example 1. Data Mining to Identify Potential Fusion Partners for Incorporation into GPCRs (comparative)

An initial screen of the Protein Data Bank (PDB; Worldwide Protein Data Bank, www.wwpdb.org) was carried out to identify potential new fusion partners that satisfy the criteria listed in Table 1 above: candidate N and C termini of potential fusion partner should be separated by no more than 15 Å; molecular weight of less than 25 kD; crystals of potential fusion partner should have diffraction resolution better than 3 Å; crystals of potential fusion partner should form in at least two different chemical conditions; and crystals of potential fusion partner should result in more than one space group. The following potential fusion partners were identified by this screen: *E. coli* flavodoxin (PDB ID:1AG9, MW: 20 kD), *M. tuberculosis* hypothetical protein, (PDB ID: 2ASF, MW: 15 kD), *E coli* CHEY (PDB ID: 1JBE, MW: 14kD), *Bos taurus* BPT1 (PDB ID: 1G6X, MW: 7 kD).

### Example 2. S1P1-Fusion Partner Proteins

S1P1 is a GPCR that binds the lipid signaling molecule sphingosine 1-phosphate (S1P), a circulating lipid that binds to five GPCRs termed S1P₁₋₅. S1P₁ selectively regulates physiological functions in the immune and cardiovascular systems, including immune cell trafficking and the maintenance of endothelial integrity. Four novel S1P1-fusion partner proteins were produced and evaluated as described below.

Each of the following proteins was evaluated as a potential fusion partner: T4 lysozyme C-terminal fragment ("Cterm-T4L" PDB ID: 2O7A, T4 Enterobacteria phage, 14 kD); flavodoxin (PDB ID: 1I1O, *Desulfovibrio vulgaris,* mutant Y98H, 16kD); Cytochrome b₅₆₂ RIL ("Bril" PDB ID: 1M6T, *Escherichia coli* soluble cytochrome b562, 12 kD); and chemotaxis protein cheA (PDB ID: 1TQG, CheA phosphotransferase domain from *Thermotoga maritima,* 11.9 kD).

Each potential fusion partner was incorporated into S1P1 as follows: cDNA constructs encoding fusion partners were synthesized encoding all amino acids observed in their respective crystal structures. To create S1P1 fusions, unique restriction sites were first introduced by PCR-based site-directed mutagenesis within the S1P1 cDNA between amino acids codons S232/R233 and K243/A244 to create a site for inserting fusion partner cDNA. Fusion partner cDNAs (*i.e.,* cDNA encoding fusion partners) were then amplified by PCR with primers that contained encoded terminal restriction sites matching the sites introduced within the S1P1 receptor DNA. cDNA for the S1P1 receptor (GenBank: M31210.1) was used as a base construct for further modification and its amino acid sequence is shown below:

### Amino acid sequence of Human S1P1 Receptor, GenBank Accession No. M31210.1

Fusion partner cDNAs were inserted into the S1P1 receptor coding sequence using standard molecular biology techniques. First, cDNA encoding fusion partners and the modified S1P1 cDNA were cleaved by restriction digestion and purified. Second, digested PCR product and vector were ligated and transformed into *E. coli.* Resultant clones were verified by DNA sequencing. In this process, restriction digestion of the S1P1 vector removed a short segment of the receptor ICL3 (S232-K243) that was subsequently replaced by the inserted fusion partner cDNAs, resulting in an insertion between S1P1 receptor residues R231 and K243. To improve behavior of receptor fusions, the S1P1 receptor C-terminus was truncated to S1P1 M325. The complete amino acid sequence for each receptor fusion construct is shown below (with the fusion partner sequence indicated in *italics*):

### Amino acid sequence of S1P1-BRIL (comparative)

### Amino acid sequence of S1P1-Flavodoxin (comparative)

### Amino acid sequence of S1P1-Xylanase (comparative)

### Amino acid sequence of S1P1-Rubredoxin

*Expression construct encoding further N- and C-terminal modifications*. Synthetic cDNA encoding the set of S1P1 receptor fusions was cloned into pFastBac modified to contain an N-terminal hemaglutinin signal sequence and a C-terminal 10 histidine tag followed by a FLAG epitope. An example of the amino acid sequence encoded by the finished S1P1-BRIL construct, with the added amino acids (italic font), is shown below

### Amino acid sequence of the expression product of the S1P1-BRIL Expression Construct (comparative)

Recombinant bacmid DNA containing S1P1 receptor fusion expression constructs were generated using standard protocols as outlined in the Invitrogen Bac-to-Bac® Baculovirus Expression System manual. Briefly, a pFastBac plasmid containing the gene of interest was transformed into DH10bac cells and transformants were color-selected on the appropriate agar plates. White colonies were selected from the transformation plates and grown in liquid culture. High-weight recombinant bacmid DNA was purified from these overnight bacterial cultures. *Spodoptera frugiperda* (Sf-9) cells were transfected with purified bacmid DNA using Fugene HD (*Promega*) and recombinant virus was harvested after 4 days. After amplification to generate high-titer stocks, Sf-9 cells were infected and harvested after 48 hours for analysis. The cell surface expression of each clone was assessed by flow cytometry using a fluorophore-labeled anti-FLAG antibody and was used as a metric to gauge proper folding and trafficking of receptor fusion proteins.

The total membrane fraction was then isolated from frozen whole cells by repeated dounce homogenization and centrifugation to remove soluble proteins and organelles.

S1P1-fusion partner proteins ("S1P1 fusion receptors") were purified from membranes by dodecyl-maltoside (DDM) extraction followed by immobilized metal affinity chromatography (IMAC) on Talon resin (Clontech) as detailed below. Frozen cellular membranes were thawed by adding room temperature lysis buffer to a total volume of 25mL. The S1P1 receptor antagonist W146 was added to a final concentration of 125 µM. The mixture was allowed to incubate at room temperature for 1 hour to ensure complete saturation of the receptor with ligand followed by addition of iodoacetamide to a concentration of 1mg/mL and an additional 1 hour incubation at 4°C. Receptor solubilization was initiated by addition of detergent and cholesteryl hemisuccunate (CHS) to achieve a final concentration of 0.5% DDM 0.1% CHS. The solubilization buffer consisted of the following mixture: 50mM Hepes pH 7.5; 800mM NaCl; 30mM Imidazole; 0.1% DDM 0.02% CHS; 125 µM W146. Solubilization was allowed to proceed for 2 hours at 4°C, after which the mixture was centrifuged at 70,000rpm for 35 minutes in a Ti70 rotor (Beckman). The supernatant was then used to resuspend 1mL of water washed Talon superflow IMAC resin, and the suspension was allowed to incubate overnight at 4°C. After incubation with the Talon resin, the suspension was poured into a 25mL BioRad disposable column the flow-through was collected and saved for later analysis. The collected Talon resin was then washed with 20 ml buffer containing the following components: 20mM Hepes pH 7.5; 500mM NaCl; 0.05% DDM; 0.01% CHS; 250 µM W146. The receptor was eluted with the same buffer supplemented with 200 mM imidizole.

Eluted proteins were analyzed by size-exclusion chromatography (SEC) followed by SDS-PAGE. Briefly, each S1P1-fusion partner protein was formulated at crystallization concentration, e.g., at 50mg/mL, diluted 30-fold and injected onto an analytical HPLC where the sample was separated using size exclusion chromatography (SEC) to obtain S1P1-fusion partner proteins of near homogeneity. The S1P1-Cterm-T4L, S1P1-flavodoxin, and S1P1-Bril S1P1-fusion partner proteins were stable in the presence or absence of ligand, and remained monodisperse even after concentration to >50 mg/mL, as indicated by traces from analysis using size exclusion chromatography (SEC) (data not shown). SEC fractions corresponding to S1P1-fusion partner proteins were evaluated separated by SDS-PAGE on a 10% gel and stained, confirming that each S1P1-fusion partner protein could be purified to near-homogeneity (data not shown).

After an initial evaluation of the expression level of each S1P1-fusion partner protein, followed by small-scale purification and SEC analysis, further work on the S1P1-cheA (PDB ID: 1TQG) protein was abandoned due to low expression levels and a poor SEC peak profile. The remaining three constructs, encoding S1P1-Cterm-T4L, S1P1-flavodoxin, and S1P1-Bril, were overexpressed and purified to near homogeneity using the protocol outlined above.

### Example 3. β2 Adrenergic Receptor Fusion Partner Proteins

A panel of candidate fusion partners was each inserted into the β2-adrenergic receptor (β2AR; b2 adrenergic receptor), and each resulting β2AR-fusion partner protein was expressed, extracted, and purified to determine various properties. β2AR fusions were constructed, expressed, and evaluated for the following β2AR-fusion partner proteins: β2AR-T4L, β2AR-C-term T4L, β2AR-Bril, β2AR-rubredoxin, β2AR-xylanase, and β2AR-flavodoxin.

Using the previously solved X-ray crystal structure of the β2 adrenergic receptor fused to T4-lysozyme, designated β2AR-T4L ("Beta-2 adrenergic receptor/T4-lysozyme chimera" PDB ID 3D4S) as a guide, candidate fusion partners were inserted into β2AR at positions that correspond to directly replacing the fused T4L sequence in the original β2AR-T4L (*i.e*., PDB ID 3D4S). Therefore, the junction sites remained identical to the original β2AR-T4L protein. All expression constructs carried an N-terminal hemagglutinin signal sequence followed by a FLAG M2 epitope. The C-terminus of the base construct was further modified to extend the original histidine purification tag from six to ten histidines to facilitate purification. All β2AR-fusion protein-encoding sequences ("receptor fusion genes") were constructed by synthetic cDNA overlap extension PCR and cloned by restriction digestion and ligation into the expression vector pFastBac1.

### Amino acid sequence of β2AR used in expression constructs (GenBank Accession No. NP_000015.1)

### Amino acid sequence of β2AR-T4L (comparative)

### Amino acid sequence of β2AR-C-term T4L (from Hanson et al., 2008) (comparative)

### Amino acid sequence of β2AR-Bril (comparative)

### Amino acid sequence of β2AR-rubredoxin

### Amino acid sequence of β2AR-xylanase (comparative)

### Amino acid sequence of β2AR-flavodoxin (comparative)

Each β2AR-fusion partner protein was extracted from the plasma membrane by incubation with a 0.5% w/v DDM/CHS detergent mixture in the presence of 0.5 mM timolol (ligand) followed by purification with immobilized metal affinity chromatography (IMAC) in the presence of 0.5 mM timolol. The same protocol was employed in the absence of ligand to test each construct for stability and favorable response to ligand binding. In the case of the ligand-bound purification the protein was subjected to secondary purification using a smaller amount of a second IMAC resin.

12.5 ml frozen cellular membranes were thawed by adding lysis buffer containing 2 mM timolol to a total volume of 25mL. The mixture was allowed to incubate at 4°C for 1 hour to ensure complete saturation of the receptor with ligand followed by addition of iodoacetamide to a concentration of 1mg/mL and an additional 1 hour incubation at 4°C. Receptor solubilization was initiated by addition of detergent and cholesteryl hemisuccinate to achieve a final concentration of 0.5% DDM 0.1% CHS. The solubilization buffer consisted of the following mixture: 50mM Hepes pH 7.5; 150 mM NaCl; 25 mM Imidazole; 0.1% DDM 0.02% CHS; 500 µM timolol. Solubilization was allowed to proceed for 2.5 hours at 4°C, after which the mixture was centrifuged at 70,000rpm for 35 minutes in a Ti70 rotor (Beckman). The supernatant was then used to resuspend 1mL of water washed Talon superflow IMAC resin, and the suspension was allowed to incubate overnight at 4°C. After incubation with the Talon resin, the suspension was poured into a 25mL BioRad disposable column the flow through was collected and saved for later analysis. The collected Talon resin was then washed with 20 mL buffer containing the following components: 50 mM Hepes pH 7.5; 500mM NaCl; 0.05% DDM; 0.01% CHS; 500 µM Timolol. The receptor was eluted with the same buffer supplemented with 200 mM imidizole. A ligand-free purification was accomplished by following the same steps, but omitting the antagonist Timolol from all buffers.

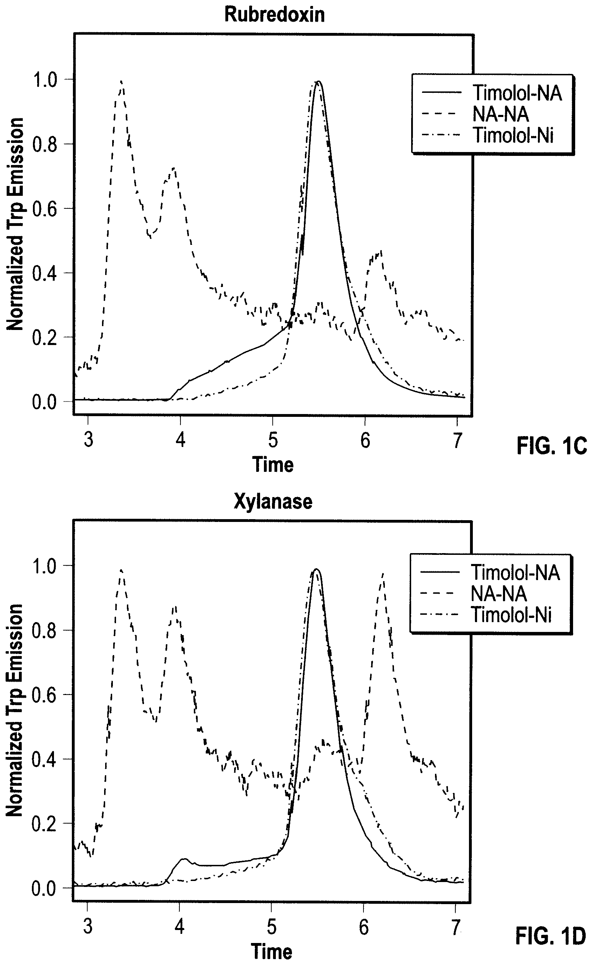

The eluate collected from the IMAC purification was injected on to an analytical SEC column and traces were monitored by UV absorbance and tryptophan fluorescence. Peak profiles were used to compare the yield and relative quality of receptor fusion proteins. Briefly the yield of functionally folded protein can by estimated by integrating the area under the peak and comparing this area to that of a reference standard with a known concentration and extinction coefficient such as bovine serum albumin. The quality of the protein is estimated based on the monodispersity of the protein SEC peak where the absence of secondary peaks or shoulders of the main peak indicate high quality (Figure 1, A-E) as well as by electrophoresis on a 10% SDS-PAGE gel with total protein staining (Figure 1F).

Figure 1 shows results and properties of each of the panel of candidate fusion partners inserted into β2AR. Figures 1A-1E shows SEC data corresponding to extraction and primary purification of each β2AR-fusion partner protein in the presence of ligand (black trace) and absence of ligand (red trace) and secondary purification of the ligand-bound species of fusion (green trace).

Figure 1F shows SDS-PAGE on a 10% gel for the final purified product of each β2AR fusion partner protein, from left to right, MW standards, β2ART4L, β2AR-C-term T4L, β2AR-Bril, β2AR-rubredoxin, β2AR-xylanase, and β2AR-flavodoxin.

Based on this initial assessment of a panel of candidate fusion partner proteins, four (4) constructs were selected for further analysis after large-scale expression and purification for their ability to crystallize in the lipidic cubic phase. Of the four (4) β2AR-fusion partner proteins that were further analyzed after large-scale expression and purification for their ability to crystallize in the lipidic cubic phase, β2AR-Bril resulted in crystal hits, *i.e.*, yielded acceptable crystals. The β2AR-Bril fusion partner protein gave initial microcrystalline hits and was optimized further to support high-resolution diffraction and structural solution. Additionally, β2AR-xylanase was successfully crystallized to form crystals that diffracted to a resolution of approximately 5 A.

Expression and purification of β2AR-Bril was carried out as described herein, with the exception of carazolol inclusion in the buffers instead of timolol, resulting in a protein sample of greater than 95% purity as assayed by SDS-PAGE. The resulting purified protein was concentrated to 60mg/mL and analyzed by analytical SEC to determine final concentration and level of heterogeneity (Figures 2A-C). The purified, concentrated material was then reconstituted into lipidic cubic phase containing 10% w/w cholesterol. Small aliquots of the reconstituted protein lipid mixture were dispensed onto a 96-well glass crystallization plate and overlayed with potential crystallization inducing buffers and reagents. Specific reagents that induce crystallization were determined resulting in large, birefringent crystals (Figure 2D) that are confirmed to be protein in nature by tryptophan fluorescence of the crystals (Figure 2E) and diffraction (Figure 2F).

### FRAP studies of β2AR-Bril fusion partner protein (comparative)

FRAP analysis of β2AR-Bril was carried out to identify alternative crystallization conditions to be explored, for the purpose of generating higher resolution crystals. β2AR-Bril was labeled with Cy3 fluorescent dye (~550 nm excitation, ~570 nm emission) Cy3 monofunctional N-hydroxysuccinimide ester (Cy3 NHS) (GE Healthcare) was added from a 5 mg/mL stock in DMF at a dye/protein molar ratio of 2-5 to purified protein bound to the second IMAC purification column in the purification protocol. The resulting solution was mixed and allowed to incubate for 2-3h at 4°C in the absence of light. After this incubation, unreacted dye was washed the column using an excess of purification buffer. The column was further washed by addition of an excess of buffer, and the column was then incubated for at least 12 hours in the dark at 4°C. After this incubation, the column with bound protein was washed with additional buffer until no evidence of unreacted dye was observed through spectrometric analysis. The labeled protein was eluted from the IMAC column, concentrated, and reconstituted into the lipidic cubic phase crystallization environment. Fluorescence recovery after photobleaching (FRAP) was used to measure two dimensional lateral diffusion of Cy3-labelled β2AR-Bril after small amounts of lipidic cubic phase β2AR-Bril ("LCP/b2bril") material was incubated with a variety of conditions thought to enable crystallization. The results of the diffusion analysis within the crystallization matrix of the lipidic cubic phase could be used to narrow down the search for crystallization space if no known crystallization conditions exist. Alternatively, FRAP analysis could be used to optimize known conditions by maximizing the percent recovery of the labeled protein and optimizing the diffusion rate to support larger crystals. For β2AR-Bril, FRAP analysis was used to identify alternative crystallization conditions to explore for generating higher resolution crystals. In some embodiments, The β2AR-Bril fusion partner protein was prepared as described above using β2AR wtN187, and the resulting β2AR-Bril was designated "IMPT-1497."

LCP-FRAP. Cy3 labeled protein for LCP-FRAP analysis was reconstituted into LCP and dispensed onto glass sandwich plates as described in the crystallization section. The setup for automated high-throughput LCP-FRAP analysis has been described. Briefly, LCP sandwich plates were loaded onto a custom built LCP-FRAP station consisting of a Zeiss AxioImager A1 fluorescent microscope, a Micropoint dye cell laser (Photonic Instruments), a cooled CCD FireWire camera CoolSnap HQ2 (Photometrics), and an automated XYZ microscope stage MS-2000 (Applied Scientific Instrumentation). Each LCP drop was then bleached by firing 15-20 laser pulses at a 25 Hz pulse rate. Fluorescence images were taken immediately before and after bleaching. After a recovery period of 30 minutes, images were taken again to determine fluorescence recovery, and analyzed by ImagePro Advanced Microscopy Suite (Media Cybernetics).

Thermostability assay. N-[4-(7-diethylamino-4-methyl-3-coumarinyl)phenyl]maleimide (CPM) dye was purchased from Invitrogen and dissolved in DMSO (Sigma) at 4 mg/mL as the stock solution for future use. The stock solution was kept at -80°C and was diluted 1:40 in dye dilution solution (10 mM buffer, 500 mM NaCl, 10% glycerol, 0.025% DDM and 0.005% CHS) before use. The thermal denaturation assay was performed with total volume of 200 µL sample in a quartz fluorometer cuvette (Starna Cells, Inc., Atascadero, CA). Receptor (4 µg) was diluted in the appropriate buffer solution to a final volume of 200 µL, 5 µL of the diluted dye was added to the protein solution and incubated for 30 min at 4°C. The mixed solution was transferred to the cuvette and the data were collected by a Cary Eclipse spectrofluorometer (Varian, USA) with a temperature ramping rate at 2°C/min. The excitation wavelength was 387 nm and the emission wavelength was 463 nm. All assays were performed over a temperature range starting from 20°C and 95°C. The stability data were processed with GraphPad Prism program (GraphPad Prism, Graphpad Software, San Diego, CA, USA). In order to determine the melting temperature (Tm), a Boltzmann sigmoidal equation was used to fit to the data.

Thermostability. The thermostability of the different β2AR constructs was then evaluated. Assays were carried out in the presence of 100 µM timolol to maintain receptor stability during heating. Relative to β2AR-T4L, β2AR-flavodoxin and β2AR-CtermT4L were less stable by about 9 °C and 4 °C, respectively. β2ARxylanase was more stable than β2AR-T4L by about 3 °C, while both β2AR-BRIL and β2AR-rubredoxin were more stable than β2AR-T4L by approximately 8 °C. We thus selected β2AR-BRIL and β2ARrubredoxin for further crystallization studies.

Crystallization. In meso crystallization methods for membrane proteins have been described in detail. Protein solution is mixed with a molten lipid mixture of 9:1 monoolein:cholesterol, at a ratio of 40% protein with 60% lipid in a custom syringe mixer. Syringe containing reconstituted LCP is loaded onto an automated crystallization robot (NT8-LCP, Formulatrix) and 35-50 nL of LCP is dispensed onto 96-well glass sandwich plates (MarienFeld), and then overlaid with 800 nL of precipitant solution. Drops are sealed with a coverslip and incubated and imaged at 20C in an automatic incubator/imager RockImager 1000 (Formulatrix). Crystals of both β2AR-BRIL and A_{2A}AR -BRIL grew within 7 days.

Protein Diffusion and Crystallization in LCP. We were able to grow crystals of β2AR-BRIL in LCP without the need for junction site optimization, as was necessary with A_{2A}AR-BRIL. LCP-FRAP assay was carried out in similar fashion to A_{2A}AR-BRIL in order to guide crystallization trials. Proteins were labeled with Cy3-mono NHS ester, in pH 7.5 buffer, and likewise evaluated for purity, monodispersity, and labeling efficiency by analytical size-exclusion chromatography (aSEC) prior to LCP-FRAP sample preparation. Labeled protein was reconstituted into LCP by mixing protein solution with molten monoolein in a final ratio of 40% (w/w) protein solution, 54% (w/w) monoolein, 6% (w/w) cholesterol, and incubated with home-made screens as introduced previously. The pH for the screens were adjusted to 6, 7, and 8 based on previous β2AR crystallization conditions. Among all three pHs, β2AR-BRIL displayed optimal mobile fractions at pH 7, which were higher than 60% with specific precipitants (supplemental fig 8a). Conditions that showed high diffusion recovery were further optimized for crystallization trails. Optimized crystals grew to approximately 80 x 15 microns and diffracted to 2.8 Å.

X-ray Data Collection. Crystallographic data were collected on the 23ID-B/D beamline (GM/CA CAT) of the Advanced Photon Source at the Argonne National Laboratory using a 10 µm collimated minibeam at a wavelength of 1.0330 Å and a MarMosaic 300 detector. To reduce radiation damage crystals were translated to a fresh position, if possible, or replaced after collecting 5-10 frames at 3 s exposure and 1° oscillation with unattenuated beam. For structure determination, datasets from 55 A2AAR crystals were integrated, scaled and merged together using HKL2000. Initial molecular replacement solution was obtained by Phaser using the A2AAR part of the A2AAR-T4L/ZM structure (PDB code 3EML) as a search model. BRIL residues were built manually in the excessive ΣA-weighted 2|Fo|-|Fc| density by repetitive cycling between Coot and Refmac5 and the resulting model was further refined using the same procedure until convergence.

Further optimization of the β2AR-Bril crystallization conditions have identified a crystallant additive (tri-methyl amine N-oxide) that results in superior crystal growth and diffraction properties (Figure 3A). When tri-methyl amine N-oxide was included, β2AR-Bril crystals diffracted x-rays to a nominal resolution of 2.8 Å (Figure 3B).

### Example 4 Adenosine A2a Receptor Fusion partner proteins With Candidate Fusion Partners

A panel of candidate fusion partners was each inserted into the adenosine A2a receptor ("A_{2A}" also referred to as A2A-Adenosine Receptor, ADORA2A, adenosine A_{2A} receptor, A2a), and each resulting A_{2A}-fusion partner protein was expressed, extracted, and purified to determine various properties.

Each A_{2A}-fusion partner construct was made by gene synthesis techniques where short oligomers of DNA were combined by overlap extension PCR. Each fusion partner for A_{2A} was subsequently sub-cloned into our standard set of expression cassettes which incorporate the Flag epitope affinity tag on the N-terminus of the receptor and a 10x histidine tag on the C-terminus of the receptor.

All proteins were expressed, harvested and purified using essentially the same protocol described above. Recombinant bacmid DNA containing A_{2A}-fusion partner protein expression constructs were generated using standard protocols as outlined in the Invitrogen Bac-to-Bac® Baculovirus Expression System manual. *Spodoptera frugiperda* (Sf-9) cells were transfected with purified bacmid DNA and recombinant virus was harvested and amplified to generate high-titer stocks. Sf-9 cells were infected and harvested after 48 hours for analysis. The total membrane fraction was then isolated from frozen whole cells. A_{2A}-fusion partner proteins (also called "A2a receptor fusions") were purified from membranes by dodecyl-maltoside (DDM) extraction followed by immobilized metal affinity chromatography (IMAC) on Talon resin (Clontech). Eluted proteins were analyzed for yield and homogeneity by electrophoresis and analytical size exclusion chromatography. Selected A_{2A}-fusion partner protein constructs that showed acceptably high expression levels were selected for further analysis of the A_{2A}-fusion partner protein in thermal denaturation assays (CPM assays) in the presence of two different ligands, ZM241385 (antagonist) and UK-432097 (agonist).

The following A_{2A}-fusion partner proteins were made and evaluated: A_{2A}-BRIL, A_{2A}-flavodoxin, A_{2A}-T4L- Cterm-T4L lysozyme fragment (T41-Cterm, also labeled as "T4L-frag" in Figure 4), A_{2A}-rubredoxin, A_{2A}-xylanase, and A_{2A}-T4L. After expression and purification, biophysical characterization of A_{2A}-fusion partner proteins was carried out as shown in Figure 4. Eluted proteins were analyzed for yield and homogeneity by electrophoresis, monodispersity and homogeneity by analytical size exclusion chromatography, and stability induction of known A_{2A} receptor ligands by thermal denaturation assays.

Figure 4A shows analysis of yield and homogeneity using a 10% SDS-PAGE gel with total protein staining for eluted purified A_{2A}-BRIL, A_{2A}-flavodoxin, A_{2A}-T4L-Cterm ("T4L-frag"), A_{2A}-rubredoxin, A_{2A}-xylanase, A_{2A}-T4L. Figure 4B shows analysis of monodispersity and homogeneity by analytical size-exclusion chromatography (aSEC) showing protein levels (UV absorption at 280 nm) for aSEC of eluted purified preparations of A_{2A}-BRIL, A_{2A}-flavodoxin, A_{2A}-T4L-Cterm (labeled "T4L-frag"), A_{2A}-rubredoxin, A_{2A}-xylanase, and A_{2A}-T4L.

In a further characterization, A_{2A}-fusion partner proteins were expressed in a 40 mL culture, expression levels were measured, and the molecular weight of each A_{2A}-fusion partner protein was estimated and correlated with the distance between the N and C terminus ("N-C distance) of the fusion partner as shown in Table 2 below. It was observed that constructs wherein the fusion partner had a shorter N-C distance, *i*.*e*. less than 10 A, showed lower expression levels for the corresponding A_{2A}-fusion partner protein.

| **Table 2.** | | |
|---|---|---|
| | MW of fusion partner (kDa) | N-C distance (A) |
| A_{2A}-BRIL (comparative) | 12 | 13.73 |
| A_{2A}-flavodoxin (comparative) | 16 | 11.06 |
| A_{2A}-Cterm-T4L lysozyme fragment (comparative) | 14 | 7.74 |
| A_{2A}-rubredoxin | 6 | 11.62 |
| A_{2A}-xylanase (comparative) | 21 | 7.22 |
| A_{2A}-T4L (control) | 18 | 9.01 |

Selected A_{2A}-fusion partner protein constructs that showed acceptably high expression levels were further analyzed in thermal denaturation assays (CPM assays) in the presence of two different ligands of the A_{2A} receptor, ZM241385 (antagonist) and UK-432097 (agonist). A_{2A}-BRIL, A_{2A}-flavodoxin, and A_{2A}-rubredoxin were selected on the basis of favorable characteristics, and A_{2A}-T4L was included as a control. Each A_{2A}-fusion partner protein was purified, and stock solutions of each A_{2A}-fusion partner protein were combined with each of the two ligands (ZM241385 (antagonist) and UK-432097 (agonist)) and analyzed for the point of thermal denaturation between 20 and 90 °C. Each A_{2A}-fusion partner protein had a representative thermal denaturation point (Tm) which varied depending on which compound was bound. Figure 4C shows the result of thermal denaturation assays for A_{2A}-BRIL, A_{2A}-flavodoxin, A_{2A}-rubredoxin, and A_{2A}-T4L in the presence of antagonist ZM241285. Figure 4D shows the results for thermal denaturation assays for A_{2A}-BRIL, A_{2A}-flavodoxin, A_{2A}-rubredoxin, and A_{2A}-T4L in the presence of agonist UK432097. In each case at least one alternate fusion partner compared favorably to A_{2A}-T4L, where A_{2A}-BRIL and A_{2A}-rubredoxin showed comparable or better stability than A_{2A}-T41 under both ligand conditions. Based on the this analysis, A_{2A}-BRIL and A_{2A}-rubredoxin were selected for large scale expression, purification and crystallization studies.

Ligand binding & downstream signaling. Ligand binding and downstream signaling assays were carried out to test the effect of the fusion domains on the functional activity of A2AAR. Radioactive ligand binding assays were carried out with both agonist UK432,097) and antagonist (ZM241385) in the presence and absence of 1 M NaCl. While the fusion domains had little effect on antagonist affinity, agonist affinity was consistently enhanced for all the A2AAR chimeras relative to WT A2AAR. For all the chimeras, agonist affinity was reduced by the presence of 1 M NaCl. cAMP accumulation assays were carried out in the presence of both ligands to test if the fusion domains inhibited downstream signaling (supplemental fig YY). With the exception of A2AARCtermT4L, the basal activity of each chimera showed minor to no response in the presence of either agonist or antagonist. A2AAR-CtermT4L was able to produce 50% signaling in the presence of agonist (100% defined as the signal produced by WT A2AAR in the presence of the corresponding ligand).

Thermostability. Due to their inherent flexibility, high thermostability is an important metric in the crystallization of GPCRs. To test the effect of the fusion domains on the protein, a fluorescence based thermostability assay was carried out. A2AAR constructs were incubated with either an antagonist, ZM241385, or an agonist, UK432,097, to stabilize the receptor prior to heating. When bound with ZM241385, A2AAR-BRIL, A2AAR-flavodoxin, and A2AAR-rubredoxin all had almost identical thermal transition temperatures with each other, about 6 °C higher than that of A2AAR-T4L. When bound with UK432,097, A2AAR-BRIL and A2AAR-rubredoxin showed similar thermal transition temperatures, while the transition temperature of A2AAR-flavodoxin was reduced by over 10 °C when compared with A2AAR-T4L. The improved thermostability of A2AAR-BRIL and A2AAR-rubredoxin in either antagonist or agonist bound conformations makes them attractive candidates for crystallization trials, as the availability of multiple highly stabilizing ligands increases the likelihood of finding a successful crystallizing condition. A2AAR-BRIL and A2AAR-rubredoxin were selected for further crystallization studies.

Junction optimization. Initial crystallization trials with either A2AAR-BRIL or A2AAR-rubredoxin in lipidic cubic phase (LCP) did not produce any crystals when the interface on the A2AAR side of the junction was chosen based on junctions originally optimized for the insertion of T4L, which has a different distance between the N- and C- termini (10.1 A) than that of BRIL (13.7 Å) and rubredoxin (11.6 Å). The secondary structure at the interface is also different between all three domains: the T4L interface consists of perpendicular α-helices, BRIL consists of anti parallel α-helices, and rubredoxin is composed of loops. These considerations led to testing of adding or removing native residues on the A2AAR side of the junction to improve the thermostability of the protein. Multiple alternative junctions that could improve the stability of both A2AAR-BRIL and A2AAR-rubredoxin were identified. Of these, the most stabilizing junctions for A2AAR-BRIL were either the removal or addition of 3 residues on the TM6 side of the junction, which increased the stability of the protein by approximately 4°C. For A2AAR-rubredoxin, the most stabilizing junctions were the addition of 2 residues on TM5 or the removal of 3 residues on TM6, both of which raised the stability of the protein by approximately 4°C.

Protein Diffusion and Crystallization in LCP. The thermostabilized A2AAR-BRIL and A2AAR-rubredoxin constructs in complex with ZM241385 were further assessed by high-throughput LCP-FRAP assay. Proteins were preferentially labeled at the N terminus with Cy3-mono NHS ester at pH 7.5 to minimize interferences to the protein core. All protein samples were evaluated for purity, monodispersity, and labeling efficiency by analytical size-exclusion chromatography (aSEC) prior to LCP-FRAP sample preparation. Labeled protein was reconstituted in LCP by mixing protein solution with molten monoolein in a final ratio of 40% (w/w) protein solution, 54% (w/w) monoolein 6% (w/w) cholesterol, and incubated with home-made screens as introduced previously. The pH for the screens were adjusted to 4, 5 and 6 based on previous A2AAR crystallization conditions. Among all three pHs, both A2AAR-BRIL and A2AAR-rubredoxin showed optimal mobile fractions at pH 5, which were higher than 70% with specific precipitants. We found that the obtained mobile fractions of A2AAR-BRIL throughout the 96-well conditions were consistently higher than that of A2AAR-rubredoxin, and proceeded with A2AAR-BRIL for further crystallization trials, with a focus on conditions that produced mobile fraction recovery higher than 70%, including citrate, tartrate, nitrate and thiocyanate. Crystallization trials were performed in 96-well glass sandwich plates (Marienfeld) by an NT8-LCP crystallization robot (Formulatrix) using 40-50 nL protein laden LCP overlaid with 0.8 µL precipitant solution in each well, and sealed with a glass coverslip (Cherezov et al. 2004; Caffrey & Cherezov, 2009). The best diffraction quality crystals were obtained within 7 days in 25-28% (v/v) PEG 400, 0.04 to 0.06 M sodium thiocyanate, 2% (v/v) 2,5-hexanediol, 100 mM sodium citrate pH 5.0. Crystals grew to an average size of 60x10x3 µm and diffracted to 1.7 Å. The high-resolution crystal structure of A2AAR-BRIL was solved at 1.8 Å.

### Example 5 Adenosine A2a Receptor Fusion partner protein conjugates with b562RIL (comparative)

A fusion partner-GPCR protein conjugate was made with adenosine receptor by replacing its third intracellular loop with apo-cytochrome b562RIL and crystals were obtained permitting elucidation of its structure at 1.8 angstrom resolution. Here we replaced the third intracellular loop (ICL3) of the human A2A adenosine receptor (A2AAR) with a thermostabilized apocytochrome b562RIL (BRIL) and determined the crystal structure of this chimeric protein (referred to as A2AAR-BRIL-ΔC) in complex with a high-affinity, subtype-selective antagonist, ZM241385, at 1.8 Å resolution (table S1).

The A2AAR-BRIL-ΔC construct in complex with ZM241385 was crystallized in a cholesterol-rich membrane-like environment of the lipidic cubic phase. The overall 1.8 Å resolution structure is nearly identical to the original crystal structure of A2AAR-T4L-ΔC/ZM241385 (PDB ID 3EML; 2.6 Å resolution), with an all-atom RMSD = 0.44 Å over 82% of A2AAR, and to the structure of a thermostabilized mutant A2AAR/ZM241385 (PDB ID 3PWH; 3.3 Å) (RMSD = 0.70 Å over 84% of A2AAR). A distal part of the extracellular loop 2 (ECL2), which was missing in all inactive state structures of A2AAR, is fully resolved. The conformations of the cytoplasmic ends of helices V and VI near the BRIL junction sites closely resemble the conformations in the A2AAR structure with unmodified ICL3, in contrast to a distorted conformation caused by the T4L fusion in A2AAR-T4L-ΔC/ZM241385.

The 1.8 Å structure includes 23 ordered lipid chains and 3 cholesterols per receptor. Together, they form an almost complete lipid bilayer around each protein molecule, mediating crystal contacts. Lipids on the extracellular side have stronger electron densities and appear to be more ordered. All three cholesterols in this structure are bound to the extracellular half of the receptor and have low B-factors (25 to 27 Å²) in comparison to other lipids (43 to 75 Å²). Two of these cholesterols (CLR1 and CLR3) are bound to symmetry-related receptors and mediate crystal lattice packing by forming face-to-face interactions. The third cholesterol molecule (CLR2) does not participate in crystal contacts. Interestingly, CLR2 and CLR3 occupy hydrophobic grooves along helix VI and form extensive contacts with the aromatic ring of Phe2556.57, which is sandwiched between them (Fig. 3C). In the adenosine family of receptors, position 6.57 is conserved as Ile, Val, or Phe: hydrophobic residues that could all support the type of stacking interaction observed in this structure. In addition, CLR2 forms a hydrogen bond (2.6 Å) with the main-chain carboxyl of Ser263, and the hydroxyl of CLR3 has a polar interaction with sulfur of Cys259 (4.0 Å) in ECL3, the loop that is stabilized by the Cys259-Cys262 disulfide bond. Specific binding and conformational stabilization of this region of helix VI by cholesterols may play a functional role in A2AAR by fixing the position of the Asn2536.55 side chain in the ligand binding pocket of the receptor. This key residue exists in all adenosine receptors and anchors the exocyclic amine of the ligand's central core in both agonists and antagonist complexes.

The A2AAR-BRIL-ΔC DNA was synthesized by GenScript with flanking restrictions sites AscI at the 5' end and HindIII at the 3' end. The gene, based on the sequences of the wild type human A2AAR and the thermostabilized apocytochrome b562 from E. coli (M7W, H102I, K106L), referred to as BRIL, included the following features: (a) residues Ala1 to Leu106 of BRIL were inserted between Lys209 to Gly218 within the A2AAR ICL3 region. (b) C-terminal residues 317-412 of A2AAR were truncated. The expression vector, designated as pFastBac1-830400, was a modified pFastBac1 vector (Invitrogen) containing an expression cassette with a BamHI flanked HA signal sequence followed by a FLAG tag at the N-terminus and with a 10xHis tag at the C-terminus. The components of the expression cassette were introduced using standard PCR based site-directed mutagenesis. The expression cassette also contained corresponding restriction sites for AscI and HindIII allowing for the standard restriction digest and subsequent ligation of the synthesized A2AAR-BRIL-ΔC DNA.

For the biochemical characterization, all constructs were digested from the pFastBac1-830400 expression vectors using BamHI and HindIII restriction enzymes. After digestion the inserts were subcloned into pcDNA3.1(-) using the endogenous restriction sites BamHI and HindIII and their sequences were verified. HEK293 cells were grown in culture medium consisting of Dulbecco's modified Eagle's medium (DMEM) supplemented with 10% newborn calf serum (NCS), 50 µg/ml streptomycin and 50 IU/ml penicillin at 37 °C and 7% CO2. Cells were subcultured twice a week at a ratio of 1:15 on 10 cm ø plates. Cells were transfected with the indicated plasmids (10 µg each) using the calcium phosphate precipitation method (40). All experiments were performed 48 h after transfection. Cell-surface Receptor Measurement and Enzyme-linked Immunosorbent Assay Twenty-four hours after transfection, cells were split into 96-well poly-D-lysine-coated plates at a density of 105 cells per well. After an additional 24 h, cell-surface receptors were labeled with mouse anti-FLAG (M2) primary antibody (Sigma, 1:1000) in culture medium for 30 min at 37°C. The cells were then washed once with DMEM supplemented with 25 mM HEPES and then incubated for another 30 min at 37 °C in culture medium supplemented with horseradish peroxidase-conjugated antimouse IgG produced in goat (Brunswig) (1:5000) as the secondary antibody. The cells were washed twice with phosphate-buffered saline (PBS). Finally, the cells were incubated with 3,3',5,5'-tetramethylbenzidine (TMB) for 5 min in the dark at room temperature. The reaction was stopped with 1M H3PO4 and after 5 min the absorbance was read at 450 nm using a VICTOR2 plate reader (PerkinElmer Life Sciences). Control experiments were performed in which no secondary or primary antibody was added. In both cases no absorbance was observed.

Competition Binding Assays using HEK293 cell membranes [3H]ZM241385 (27.4 Ci/mmol) was obtained from ARC Inc. (St. Louis, MO, USA). ZM241385 and CGS21680 were obtained from Ascent Scientific (Bristol, UK), while UK432,097 was obtained from Axon (Groningen, The Netherlands). All other materials were purchased from commercial sources and were of the highest available purity. HEK293 cells were grown and transfected as described above. Membranes were prepared as follows. Cells were detached from plates 48 h after transfection by scraping them into 5 ml PBS, collected and centrifuged at 700 ×g (3000 r.p.m.) for 5 min. Pellets derived from 8 plates (10 cm ø) were pooled and resuspended in 8 ml of ice-cold 50 mM Tris-HCl buffer with 5 mM MgCl2, pH 7.4. An UltraThurrax was used to homogenize the cell suspension. Membranes and the cytosolic fraction were separated by centrifugation at 100,000 ×g (31,000 r.p.m.) in a Beckman Optima LE-80K ultracentrifuge at 4 °C for 20 min. The pellet was resuspended in 4 ml of Tris buffer and the homogenization and centrifugation step was repeated. Tris buffer (2 ml) was used to resuspend the pellet and adenosine deaminase (ADA) was added (0.8 IU/ml) to break down endogenous adenosine. Membranes were stored in 250 µL aliquots at -80 °C. Membrane protein concentrations were measured using the BCA (bicinchoninic acid) method. For competition binding experiments, 25 µg of membranes were used at first for the single point experiments, while subsequently between 6 and 20 µg of protein was used for the experiments with whole curves to ensure that total binding was less than 10% of the total radioactivity added to prevent radioligand depletion. Membrane aliquots were incubated in a total volume of 100 µl of assay buffer (50 mM Tris-HCl, pH 7.4, supplemented with 5 mM MgCl2) at 25 °C for 2 h. Radioligand displacement experiments were performed using five concentrations of competing ligand (ZM241385 or UK432,097) in the absence and presence 1M NaCl. [3H]ZM241385 was used at a concentration of ~ 4.0 nM. Nonspecific binding was determined in the presence of 100 µM CGS21680 and represented less than 10% of the total binding. Incubations were terminated by rapid vacuum filtration to separate the bound and free radioligand through 96-well GF/B filter plates using a Filtermate-harvester (PerkinElmer Life Sciences). Filters were subsequently washed three times with ice-cold wash buffer (50 mM Tris HCl supplemented with 5 mM MgCl2, pH 7.4). The filter-bound radioactivity was determined by scintillation spectrometry using the PE 1450 Microbeta Wallac Trilux scintillation counter (PerkinElmer Life Sciences).

Demonstration of downstream signaling by intracellular cAMP determination. HEK293T cells were grown and transfected as described above. Cells were harvested 48 h after transfection and the amount of cAMP produced was determined with the LANCE Ultra cAMP 384 kit following the recommended protocol (PerkinElmer Life Sciences). In short, 4000 cells/well were pre-incubated with single concentrations of either UK432,097 (30 nM, approx. EC80) in the absence or presence of ZM241385 (100 nM, approx. 100 × Ki) or ZM241385 (100 nM) alone for 30 min at 37 °C in stimulation buffer (PBS supplemented with 5 mM HEPES, 0.1% BSA, 50 µM rolipram, 50 µM cilostamide and 0.8 IU/ml ADA). Subsequently, detection and antibody solutions were added according to manufacturer's instructions, which were followed by an incubation of 1 hr at room temperature in the dark. The generated fluorescence intensity was quantified on the EnVision® Multilabel Reader (PerkinElmer, Groningen, Netherlands).

Receptor expression in Sf9 cells and purification. High-titer recombinant baculovirus (>108 viral particles per ml) was obtained using the Bac-to-Bac Baculovirus Expression System (Invitrogen). Briefly, recombinant baculoviruses were generated by transfecting 5 µg of recombinant bacmid containing the target gene sequence into Spodoptera frugiperda (Sf9) cells using 3 µl of FuGENE HD Transfection Reagent (Roche) and Transfection Medium (Expression Systems). Cell suspension was incubated for 4 d while shaking at 27 °C. P0 viral stock was isolated after 4 d and used to produce high-titer baculovirus stock. Viral titers were performed by flow-cytometric method after staining cells with gp64-PE (Expression Systems) (42). Sf9 cells at a cell density of 2-3 × 106 cells/ml were infected with P2 virus at MOI (multiplicity of infection) of 3. Cells were harvested by centrifugation at 48 h post infection and stored at -80 °C until use. Insect cell membranes were disrupted by thawing frozen cell pellets in a hypotonic buffer containing 10 mM HEPES (pH 7.5), 10 mM MgCl₂, 20 mM KCl and an EDTA-free complete protease inhibitor cocktail (Roche). Extensive washing of the isolated raw membranes was performed by repeated dounce homogenization and centrifugation in the same hypotonic buffer (∼2-3 times), and then in a high osmotic buffer containing 1.0 M NaCl, 10 mM HEPES (pH 7.5), 10 mM MgCl₂, 20 mM KCl (~3-4 times) to remove soluble and membrane associated proteins. Purified membranes were resuspended in 10 mM HEPES (pH 7.5), 10 mM MgCl₂, 20 mM KCl, and 40% glycerol, flash-frozen in liquid nitrogen and stored at -80 °C until further use. Prior to solubilization, purified membranes were thawed on ice in the presence of 4 mM theophylline (Sigma), 2.0 mg/ml iodoacetamide (Sigma), and an EDTA-free complete protease inhibitor cocktail (Roche). After incubation for 30 min at 4 °C, membranes were solubilized by incubation in the presence of 0.5% (w/v) n-dodecyl-β-D-maltopyranoside (DDM) (Anatrace) and 0.1% (w/v) cholesteryl hemisuccinate (CHS) (Sigma) for 2.5-4 h at 4 °C. The unsolubilized material was removed by centrifugation at 150,000 ×g for 45 min. The supernatant was incubated with TALON IMAC resin (Clontech) in the buffer containing 50 mM HEPES (pH 7.5), 800 mM NaCl, 0.5% (w/v) DDM, 0.1% (w/v) CHS, and 20 mM imidazole. After overnight binding, the resin was washed with ten column volumes of 50 mM HEPES (pH 7.5), 800 mM NaCl, 10% (v/v) glycerol, 25 mM imidazole, 0.1% (w/v) DDM, 0.02% (w/v) CHS, 10 mM MgCl2, 8 mM ATP (Sigma) and 25 µM ZM241385 (Tocris, prepared as 100 mM stock in DMSO), followed by four column volumes of 50 mM HEPES (pH 7.5), 800 mM NaCl, 10% (v/v) glycerol, 50 mM imidazole, 0.05% (w/v) DDM, 0.01% (w/v) CHS and 25 µM ZM241385. The receptor was eluted with 25 mM HEPES (pH 7.5), 800 mM NaCl, 10% (v/v) glycerol, 220 mM imidazole, 0.025% (w/v) DDM, 0.005% (w/v) CHS and 25 µM ZM241385 in a minimal volume. Purified receptor in the presence of ZM241385 was concentrated from ∼0.4 mg/ml to 60 mg/ml with a 100 kDa molecular weight cut-off Vivaspin concentrator (GE Healthcare). Receptor purity and monodispersity was followed using SDS-PAGE and analytical size exclusion chromatography (aSEC).

Crystallization. Protein samples of A2AAR-BRIL-ΔC in complex with ZM241385 were reconstituted into lipidic cubic phase (LCP) by mixing with molten lipid using a mechanical syringe mixer. The protein-LCP mixture contained 40% (w/w) protein solution, 54% (w/w) monoolein (Sigma) and 6% (w/w) cholesterol (AvantiPolar Lipids). Crystallization trials were performed in 96-well glass sandwich plates (Marienfeld) by an NT8-LCP crystallization robot (Formulatrix) using 40-50 nl protein-laden LCP overlaid with 0.8 µl precipitant solution in each well, and sealed with a glass coverslip. Protein reconstitution in LCP and crystallization trials were carried out at room temperature (~21-23 °C). The crystallization plates were stored and imaged in an incubator/imager (Rocklmager 1000, Formulatrix) at 20 °C. Diffraction quality crystals of an average size of 60×10×3 µm were obtained within 7 days in 25-28% (v/v) PEG 400, 0.04 to 0.06 M sodium thiocyanate, 2% (v/v) 2,5-hexanediol, 100 mM sodium citrate pH 5.0. Crystals were harvested directly from LCP using 50 µm MiTeGen micromounts and immediately flash frozen in liquid nitrogen without adding an extra cryoprotectant.

X-ray data collection and processing. Crystallographic data were collected on the 23ID-B/D beamline (GM/CA CAT) of the Advanced Photon Source at the Argonne National Laboratory using a 10 µm collimated minibeam at a wavelength of 1.0330 Å and a MarMosaic 300 detector. To reduce radiation damage crystals were translated to a fresh position, if possible, or replaced after collecting 5 frames at 3 s exposure and 0.5° oscillation with an unattenuated beam. Datasets from 55 crystals were integrated, scaled and merged together using HKL2000.

Structure determination and refinement. Initial molecular replacement solution was obtained by Phaser using the A2AAR domain of the A2AAR-T4L-ΔC/ZM structure (PDB ID 3EML) as a search model. BRIL residues were built manually in the excessive ΣA-weighted 2|Fo|-|Fc| density by repetitive cycling between Coot and Refmac5 and the resulting model was further refined using the same procedure until convergence. Excellent electron densities observed for all cholesterols allowed for reliable distinction of this type of molecule from other protein-bound lipids and for their confident placements and orientations (Fig. 3C,D). Electron densities for other lipids, however, were not sufficiently clear to unambiguously identify their headgroups. Therefore, most of the elongated electron density tubes near the protein hydrophobic surface were modeled as oleic acids (OLA), with the exception of few that were better fit with monooleins (OLC), the major lipid component used for crystallization. The data collection and refinement statistics are shown in Table S1.

**Table S1. X-ray data collection and refinement statistics.**

| Structure | | A2AAR-BRIL-ΔC |
|---|---|---|
| Data collection | | |
| Number of crystals | | 55 |
| Space group | | *C2221* |
| Cell dimensions | | |
| a, b, c (Å) | | |
| | | 39.44, 179.52, 140.31 |
| Number of reflections measured | | 176,392 |
| Number of unique reflections | | 44,413 |
| Resolution (Å) | | 29.73 - 1.80 (1.86 - 1.80) |
| Rmerge | | 0.10(0.81) |
| Mean I/σ(I) | | 17.7 (1.8) |
| Completeness (%) | | 95.1 (92.8) |
| Redundancy | | 4.0 (3.3) |
| Refinement | | |
| Resolution (Å) | | 29.73 - 1.80 |
| Number of reflections (test set) | | 2,032 (2,222) |
| Rwork / Rfree | | 0.18 / 0.22 |
| Number of atoms | | |
| Proteins | | 3,137 |
| Ligand (ZM241385) | | 25 |
| Lipids | | 447 |
| Na+ | | 1 |
| Other | | 189 |
| | | |
| Average *B* value (Å²) | | |
| A2AAR | | 22.8 |
| BRIL | | 48.0 |
| Ligand (ZM241385) | | 18.6 |
| Lipids | | 52.1 |
| Na+ | | 26.2 |
| Other | | 38.8 |
| R.m.s. deviations | | |
| Bond lengths (Å) | | 0.014 |
| Bond angles (°) | | 1.54 |
| Ramachandran plot statistics (%)* | | |
| Most favored regions | | 99.5 |
| Additionally allowed regions | | 0.5 |
| Disallowed regions | | 0.0 |

| | | |
|---|---|---|
| * As defined in MolProbity. | | |

### Example 6. Opioid Receptor Fusion partner proteins With Candidate Fusion Partners

A panel of candidate fusion partners was each inserted into the opioid receptor 1 ("NOP1") each resulting NOP1-fusion partner protein was expressed, extracted, and purified to determine various properties. NOP1 was used to prepare fusion partner proteins with fusion partners Bril (BRIL, bRIL), flavodoxin, Cterm-T4L, rubredoxin, xylanase, and T4L (as control) for studies described below. Fusion partner proteins with Bril, flavodoxin, Cterm T4L and xylanase are comparative examples.

Each NOP1-fusion partner construct was made by gene synthesis techniques where short oligomers of DNA were combined by overlap extension PCR. Each fusion partner for NOP1 was subsequently sub-cloned into our standard set of expression cassettes which incorporate the Flag epitope affinity tag on the N-terminus of the receptor and a 10x histidine tag on the C-terminus of the receptor.

Preliminary analysis of expression results indicated that the NOP1-flavodoxin fusion partner protein ("NOP1-flavodoxin") yielded the highest levels of expression. NOP1-BRIL had the second-highest expression level.

All fusion proteins were expressed, harvested and purified using essentially the same protocol. Recombinant bacmid DNA containing NOP1 receptor fusion expression constructs were generated using standard protocols as outlined in the Invitrogen Bac-to-Bac® Baculovirus Expression System manual. *Spodoptena frugiperda* (Sf-9) cells were transfected with purified bacmid DNA and recombinant virus was harvested and amplified to generate high-titer stocks. Sf-9 cells were infected and harvested after 48 hours for analysis. The total membrane fraction was then isolated from frozen whole cells. NOP1 fusion partner proteins were purified from membranes by dodecyl-maltoside (DDM) extraction followed by immobilized metal affinity chromatography (IMAC) on Talon resin (Clontech). Eluted proteins were analyzed for yield and homogeneity by electrophoresis on a 10% SDS-PAGE gel using a western blot employing antibodies directed toward the Flag epitope on the N-terminus of the receptor (Figure 6A) as well as for monodispersity using analytical size-exclusion chromatography (aSEC) (Figure 6B).

Analysis of the SDS-PAGE gel with western blot using an anti-FLAG antibody, and analytical SEC, indicate that insertion of full-length T4L as a fusion partner results in significantly less expression of the construct (the construct encoding NOP1-T4L) compared to wild-type NOP1, as well as poor monodispersity, which are both indicative of a lower stability construct. In contrast, the NOP1-flavodoxin and NOP1-bRIL constructs have significantly greater expression levels (Figure 6A) and improved monodispersity (Figure 6B).

Constructs were analyzed in thermal denaturation assays to determine thermal transition temperatures, as shown in Figure 7. Comparison of full-length NOP1-T4L control to the alternate fusion proteins NOP1-flavodoxin and NOP1-bRIL indicates a greater stability for both the flavodoxin and bRIL alternate fusion partners (Figure 7A). Full-length T4L as a fusion partner appears to decreases the Tm relative to wild-type by 10 °C regardless of junction position at which T4L is inserted into NOP1 (Figure 7B), whereas in contrast, neither NOP1-flavodoxin nor NOP1-bRIL had a negative effect on the transition temperature (Figure 7A).

*Junction optimization* It was considered that initial estimates for optimal placement of the fusion partner within the context of the third intracellular loop are often not ideal, for example when pursuing a new receptor target such as NOP1. Based on the initial expression and aSEC results, flavodoxin was selected as a good candidate for exploring optimization of the junction site between the receptor and the fusion protein. Based on SDS-PAGE analysis coupled with western blotting of the N-terminal Flag epitope it was found that optimal flavodoxin placement could be detected by the disappearance of high molecular weight oligomeric species which are often indicative of poor stability (Figure 7A).

### Example 7. Chemokine CCR5 Receptor Fusion Partner Proteins

The chemokine receptor CCR5 was used to prepare fusion partner proteins with fusion partners Bril (cytB, BRIL, bRIL), flavodoxin, Cterm-T4L, rubredoxin, xylanase, and T4L (as control) for studies described below.

Fusion partner proteins with Bril, flavodoxin, Cterm T4L and xylanase are comparative examples.

Each CCR5-fusion partner construct was made by gene synthesis techniques where short oligomers of DNA were combined by overlap extension PCR. Each fusion partner for CCR5 was subsequently sub-cloned into our standard set of expression cassettes which incorporate the Flag epitope affinity tag on the N-terminus of the receptor and a 10x histidine tag on the C-terminus of the receptor.

Preliminary analysis of expression results indicated that CCR5-rubredoxin and CCRS-Cterm-T4L yielded the highest levels of expression (Figure 8A).

All fusion proteins were expressed, harvested and purified using essentially the same protocol. Recombinant bacmid DNA containing CCR5 receptor fusion expression constructs were generated using standard protocols as outlined in the Invitrogen Bac-to-Bac® Baculovirus Expression System manual. *Spodoptera frugiperda* (Sf-9) cells were transfected with purified bacmid DNA and recombinant virus was harvested and amplified to generate high-titer stocks. Sf-9 cells were infected and harvested after 48 hours for analysis. The total membrane fraction was then isolated from frozen whole cells. CCR5 fusion partner proteins were purified from membranes by dodecyl-maltoside (DDM) extraction followed by immobilized metal affinity chromatography (IMAC) on Talon resin (Clontech). Eluted proteins were analyzed for yield and homogeneity by electrophoresis on a 10% SDS-PAGE gel using a western blot employing antibodies directed toward the Flag epitope on the N-terminus of the receptor (Figure 8A) as well as for monodispersity using analytical size-exclusion chromatography (aSEC) (Figure 8B).

Analysis of the SDS-PAGE gel with western blot employing antibodies directed toward the Flag epitope on the N-terminus of the receptor, and analytical SEC indicate that insertion of full-length T4L results in significantly less expression compared to wild-type, as well as poor monodispersity, which are both indicative of a lower stability construct. In contrast, the CCR5-rubredoxin and CCR5-cterm-T4L proteins have significantly greater expression levels (Figure 8A) and improved monodispersity (Figure 8B).

### Example 8 Nociceptin/Orphanin FQ peptide receptor Fusiopn partner protein conjugated with BRIL (comparative)

The crystal structure of nociceptin/orphanin FQ (N/OFQ) peptide (NOP) receptor was solved in complex with the small molecule antagonist from Banyu Compound-24 (C-24), revealing atomic details of ligand receptor recognition and selectivity. C-24 mimics the first four N-terminal residues of the NOP selective peptide antagonist UFP-101, a close derivative of N/OFQ. The N-terminus of hNOP was replaced with thermostabilised apocytochrome b562RIL (BRIL), and the X-ray crystal structure of this receptor-fusion in complex with Compound-24 (C-24) was determined. C-24 was selected for co-crystallization with the hNOP-BRIL construct based on the thermostability it imparts to the receptor. The BRIL-ΔN - hNOP-ΔC fusion protein was also successfully crystallized in the presence of other ligands.

**BRIL-ΔN-hNOP-ΔC.** Consistent with membrane proteins grown in lipidic cubic phase (LCP), the BRIL-ΔN -hNOP-ΔC fusion protein forms a layered type I crystal packing lattice. With two antiparallel receptor molecules in the asymmetric unit of the P21 lattice, one of the BRIL domains is disordered whereas the second forms crystal lattice contacts with two receptors from an adjacent layer. Electron density for the two receptors was excellent with low B-factors when compared to the resolved BRIL fusion domain (hNOP:A = 52.7, hNOP:B = 52.3 versus BRIL = 82.4 Å²). The transmembrane (TM) cores of the two hNOP molecules are nearly identical with a Cα RMSD of 0.6 Å.
1. A composition comprising a GPCR-fusion partner protein which comprises, from N-terminus to C terminus: (i) a first domain comprising a portion of a G-protein-coupled receptor (GPCR) wherein the first domain comprises the first through fifth transmembrane domains of the GPCR, (ii) a second domain comprising a fusion partner amino acid sequence wherein said sequence is at least 90% homologous to a protein selected from the list consisting of rubredoxin, cytochrome b₅₆₂ RIL (Bril), flavodoxin, xylanase, or a C-terminal fragment of T4lysozyme, and (iii) a third domain comprising a portion of the GPCR wherein the third domain comprises the sixth and seventh transmembrane domains of the GPCR.
2. A composition comprising a GPCR-fusion partner protein which comprises, from N-terminus to C terminus: (i) a first domain comprising a G-protein-coupled receptor (GPCR), and (ii) a second domain comprising a fusion partner amino acid sequence wherein said sequence is at least 90% homologous to a protein selected from the list consisting of rubredoxin, cytochrome b₅₆₂ RIL (Bril), flavodoxin, xylanase, or a C-terminal fragment of T4lysozyme.
3. A composition comprising a GPCR-fusion partner protein which comprises, from N-terminus to C terminus: (i) a first domain comprising a fusion partner amino acid sequence wherein said sequence is at least 90% homologous to a protein selected from the list consisting of rubredoxin, cytochrome b₅₆₂ RIL (Bril), flavodoxin, xylanase, or a C-terminal fragment of T4lysozyme, and (ii) a second domain comprising a GPCR.
4. A composition comprising a GPCR-fusion partner protein which comprises, from N-terminus to C terminus: (i) a first domain comprising a portion of a G-protein-coupled receptor (GPCR) wherein the first domain comprises the first through fifth transmembrane domains of the GPCR, (ii) a second domain comprising a fusion partner amino acid sequence wherein said sequence is at least 90% homologous to a protein selected from the list consisting of 2rhf, 2ehs, 2ip6, 3i7m, 1x3o, 1u84, 1h75, 2huj, lysq, 31s0, 2qr3, 1zuh, 2b8i, 2cgq, 3fxh, 3nph, 2o4d, 1tmy, 1vku, and 2es9, and (iii) a third domain comprising a portion of the GPCR wherein the third domain comprises the sixth and seventh transmembrane domains of the GPCR.
5. A composition comprising a GPCR-fusion partner protein which comprises, from N-terminus to C terminus: (i) a first domain comprising a G-protein-coupled receptor (GPCR), and (ii) a second domain comprising a fusion partner amino acid sequence wherein said sequence is at least 90% homologous to a protein selected from the list consisting of 2rhf, 2ehs, 2ip6, 3i7m, lx3o, 1u84, 1h75, 2huj, lysq, 31s0, 2qr3, 1zuh, 2b8i, 2cgq, 3fxh, 3nph, 2o4d, 1tmy, 1vku, and 2es9.
6. A composition comprising a GPCR-fusion partner protein which comprises, from N-terminus to C terminus: (i) a first domain comprising a fusion partner amino acid sequence wherein said sequence is at least 90% homologous to a protein selected from the list consisting of 2rhf, 2ehs, 2ip6, 3i7m, 1x3o, 1u84, 1h75, 2huj, 1ysq, 3ls0, 2qr3, 1zuh, 2b8i, 2cgq, 3fxh, 3nph, 2o4d, 1tmy, 1vku, and 2es9, and (ii) a second domain comprising a GPCR.
7. The composition of clauses 1-6 wherein the GPCR-fusion partner protein is in crystalline form.
8. The composition of clause 7 wherein the GPCR-fusion partner protein is of sufficient quality to support x-ray crystallographic structure determination for the GPCR at a resolution of at least 3 angstroms.
9. The composition of clause 1 or 4 wherein the fusion partner substantially comprises the domain corresponding to the third intracellular domain of the GPCR between the fifth and sixth transmembrane domains of the GPCR.
10. The composition of clauses 1-9 wherein the fusion partner amino acid sequence has least 95 % homology with rubredoxin.
11. The composition of clauses 1-9 wherein the fusion partner amino acid sequence has at least 95 % homology with cytochrome b₅₆₂ RIL (Bril).
12. The composition of clauses 1-9 wherein the fusion partner amino acid sequence has at least 95 % homology with flavodoxin.
13. The composition of clauses 1-9 wherein the fusion partner amino acid sequence has at least 95 % homology with xylanase.
14. The composition of clauses 1-9 wherein the GPCR is a naturally occurring GPCR.
15. The composition of clauses 1-9 wherein the GPCR is a variant of a naturally occurring GPCR.
16. The composition of clauses 1-9 wherein the GPCR is a class A GPCR.
17. The composition of clauses 1-9 wherein the GPCR is an S1P1 receptor.
18. The composition of clauses 1-9 wherein the GPCR is a β2-adrenergic receptor (β2AR).
19. The composition of clause 1-6, wherein the fusion partner is selected from rubredoxin, cytochrome b₅₆₂ RIL (Bril), flavodoxin, or xylanase.
20. The composition of clause 18, wherein the GPCR-fusion partner protein is β2AR-Bril.
21. The composition of clauses 1-9 wherein the GPCR is an adenosine A2a receptor
22. The composition of clause 21, wherein the fusion partner is selected from rubredoxin, cytochrome b₅₆₂ RIL (Bril), flavodoxin, or xylanase.
23. The composition of clauses 1-9 wherein the GPCR is an NOP1 receptor
24. The composition of clause 23, wherein the fusion partner is selected from rubredoxin, cytochrome b₅₆₂ RIL (Bril), flavodoxin, or xylanase.
25. The composition of clauses 1-9 wherein the GPCR is a CCR5 receptor.
26. The composition of clause 25, wherein the fusion partner is selected from rubredoxin, cytochrome b₅₆₂ RIL (Bril), flavodoxin, or xylanase.
27. The composition of clauses 1-26 wherein the composition further comprises tri-methyl amine N-oxide as a crystallant additive.
28. A method of using a fusion partner to support crystallization of a protein suitable for crystallographic structural studies of a G-protein-coupled receptor (GPCR) comprising:
   (a) incorporating a fusion partner into an intracellular domain of the GPCR to form a GPCR-fusion partner protein, wherein the fusion partner comprises an amino acid sequence which is at least 90% homologous with the amino acid sequence of a protein selected from the group consisting of rubredoxin, cytochrome b₅₆₂ RIL (Bril), flavodoxin, and xylanase;
   (b) expressing and purifying the GPCR-fusion partner protein; and
   (c) crystallizing the purified GPCR-fusion partner protein.
29. A method of using a fusion partner to support crystallization of a protein suitable for crystallographic structural studies of a G-protein-coupled receptor (GPCR) comprising:
   (a) attaching a fusion partner to an N-terminus or C-terminus of the GPCR to form a GPCR-fusion partner protein, wherein the fusion partner comprises an amino acid sequence which is at least 90% homologous with the amino acid sequence of a protein selected from the group consisting of rubredoxin, cytochrome b₅₆₂ RIL (Bril), flavodoxin, and xylanase;
   (b) expressing and purifying the GPCR-fusion partner protein; and
   (c) crystallizing the purified GPCR-fusion partner protein.
30. The method of clauses 28- 29, wherein the GPCR is a class A GPCR.
31. The method of clause 30, wherein the GPCR is selected from a β2-adrenergic receptor (β2AR), an A2A-adenosine receptor, an S1P1 receptor, an OLR1 receptor, or a CCR5 receptor.
32. The method of clauses 28-29, wherein the fusion partner comprises the amino acid sequence of rubredoxin.
33. The method of clauses 28-29, wherein the fusion partner comprises the amino acid sequence of cytochrome b₅₆₂ RIL (Bril).
34. The method of clauses 28-29, wherein the fusion partner comprises the amino acid sequence of T4 lysozyme C-terminal fragment (Cterm-T4L).
35. The method of clauses 28-29, wherein the fusion partner comprises the amino acid sequence of flavodoxin.
36. The method of clauses 28-29, wherein the fusion partner comprises the amino acid sequence of xylanase.
37. The method of clauses 28-29, wherein the fusion partner is incorporated in the intracellular domain between TM5 and TM 6 regions of the GPCR.
38. The method of clauses 28-37, wherein the method further comprises the step of crystallizing the purified GPCR-fusion partner protein is performed in the presence of a crystallant additive.
39. The method of clause 38 wherein the crystallant additive is tri-methyl amine N-oxide.
40. A method of selecting a candidate fusion partner for use in a fusion protein of a GPCR protein and such fusion partner where such resulting fusion protein supports formation of diffraction quality crystals comprising the steps of (a) providing one or more databases having information relating to proteins, (b) searching such one or more databases for proteins meeting the following criteria: (i) having N and C termini separated by no more than 15 Å; (ii) having a molecular weight of less than 25kD; (iii) having been demonstrated to be crystallized with a diffraction resolution of at least 3 Å; (iv) having the capacity to form crystals in more than one set of chemical conditions; and (v) having the capacity to form crystals having more than one space group; and (c) selecting as a candidate fusion partner one or more proteins satisfying criteria (i) -(v).
41. The method of clause 40 wherein in step (b) the one or more databases are searched for proteins which also meet the following criteria: (vi) having at least 50% alpha-helical content; and (vii) having alpha-helical domains at the N-terminus, or the C-terminus or both; and in step (c) candidate fusion partners are selected which satisfy criteria (i) -(vii).

## Claims

1. A composition comprising a GPCR-fusion partner protein which comprises, from N-terminus to C terminus: (i) a first domain comprising a portion of a G-protein-coupled receptor (GPCR) wherein the first domain comprises the first through fifth transmembrane domains of the GPCR, (ii) a second domain comprising a fusion partner amino acid sequence wherein said sequence is at least 90% homologous to rubredoxin, and (iii) a third domain comprising a portion of the GPCR wherein the third domain comprises the sixth and seventh transmembrane domains of the GPCR.

2. A composition comprising a GPCR-fusion partner protein which comprises, from N-terminus to C terminus: (i) a first domain comprising a G-protein-coupled receptor (GPCR), and (ii) a second domain comprising a fusion partner amino acid sequence wherein said sequence is at least 90% homologous to rubredoxin.

3. A composition comprising a GPCR-fusion partner protein which comprises, from N-terminus to C terminus: (i) a first domain comprising a fusion partner amino acid sequence wherein said sequence is at least 90% homologous to rubredoxin, and (ii) a second domain comprising a GPCR.

4. The composition of claims 1-3 wherein the GPCR-fusion partner protein is in crystalline form.

5. The composition of claim 4 wherein the GPCR-fusion partner protein is of sufficient quality to support x-ray crystallographic structure determination for the GPCR at a resolution of at least 3 angstroms.

6. The composition of claim 1 wherein the fusion partner comprises the domain corresponding to the third intracellular domain of the GPCR between the fifth and sixth transmembrane domains of the GPCR.

7. A method of using a fusion partner to support crystallization of a protein suitable for crystallographic structural studies of a G-protein-coupled receptor (GPCR) comprising:
(a) incorporating a fusion partner into an intracellular domain of the GPCR to form a GPCR-fusion partner protein, wherein the fusion partner comprises an amino acid sequence which is at least 90% homologous with the amino acid sequence of rubredoxin;
(b) expressing and purifying the GPCR-fusion partner protein; and
(c) crystallizing the purified GPCR-fusion partner protein.

8. A method of using a fusion partner to support crystallization of a protein suitable for crystallographic structural studies of a G-protein-coupled receptor (GPCR) comprising:
(a) attaching a fusion partner to an N-terminus or C-terminus of the GPCR to form a GPCR-fusion partner protein, wherein the fusion partner comprises an amino acid sequence which is at least 90% homologous with the amino acid sequence of rubredoxin;
(b) expressing and purifying the GPCR-fusion partner protein; and
(c) crystallizing the purified GPCR-fusion partner protein.

9. The method of claims 7-8, wherein the GPCR is a class A GPCR.

10. The method of claims 7-9, wherein the method further comprises the step of crystallizing the purified GPCR-fusion partner protein is performed in the presence of a crystallant additive.

## Patentansprüche

1. Eine Zusammensetzung, die ein GPCR-Fusionspartner-Protein umfasst, das vom N-Terminus bis zum C-Terminus:
I) eine erste Domäne, die einen Teil eines G-Protein-gekoppelten Rezeptors (GPCR) umfasst, wobei die erste Domäne die erste bis fünfte Transmembrandomäne des GPCR umfasst,
II) eine zweite Domäne, die eine Fusionspartner-Aminosäuresequenz umfasst, wobei diese Sequenz zu mindestens 90 % mit Rubredoxin homolog ist, und
III) eine dritte Domäne, die einen Teil des GPCR umfasst, wobei die dritte Domäne die sechste und siebente Transmembrandomäne des GPCR umfasst,
umfasst.

2. Eine Zusammensetzung, die ein GPCR-Fusionspartner-Protein umfasst, das vom N-Terminus bis zum C-Terminus:
I) eine erste Domäne, die einen G-Protein-gekoppelten Rezeptor (GPCR) umfasst, und
II) eine zweite Domäne, die eine Fusionspartner-Aminosäuresequenz umfasst, wobei diese Sequenz zu mindestens 90 % mit Rubredoxin homolog ist,
umfasst.

3. Eine Zusammensetzung, die ein GPCR-Fusionspartner-Protein umfasst, das vom N-Terminus bis zum C-Terminus:
I) eine erste Domäne, die eine Fusionspartner-Aminosäuresequenz umfasst, wobei diese Sequenz zu mindestens 90 % mit Rubredoxin homolog ist, und
II) eine einen GPCR umfassende zweite Domäne
umfasst.

4. Die Zusammensetzung nach den Ansprüchen 1 bis 3, wobei das GPCR-Fusionspartner-Protein in kristalliner Form vorliegt.

5. Die Zusammensetzung nach Anspruch 4, wobei die Qualität des GPCR-Fusionspartner-Proteins ausreicht, um eine kristallographische Röntgenstrukturanalyse des GPCR mit einer Auflösung von mindestens 3 Ängström zu erleichtern.

6. Die Zusammensetzung nach Anspruch 1, wobei der Fusionspartner die Domäne umfasst, die der dritten intrazellulären Domäne des GPCR zwischen der fünften und sechsten Transmembrandomäne des GPCR entspricht.

7. Ein Verfahren zur Verwendung eines Fusionspartners, um die Kristallisation eines Proteins zu erleichtern, das für kristallographische Strukturanalysen eines G-Protein-gekoppelten Rezeptors (GPCR) geeignet ist, welches das:
a) Einbauen eines Fusionspartners in eine intrazelluläre Domäne des GPCR, um ein GPCR-Fusionspartner-Protein zu bilden, wobei der Fusionspartner eine Aminosäuresequenz umfasst, die zu mindestens 90 % mit der Aminosäuresequenz des Rubredoxins homolog ist,
b) Exprimieren und Aufreinigen des GPCR-Fusionspartner-Proteins und
c) Kristallisieren des aufgereinigten GPCR-Fusionspartner-Proteins
umfasst.

8. Ein Verfahren zur Verwendung eines Fusionspartners, um die Kristallisation eines Proteins zu erleichtern, das für kristallographische Strukturanalysen eines G-Protein-gekoppelten Rezeptors (GPCR) geeignet ist, welches das:
a) Binden eines Fusionspartners an einen N-Terminus oder C-Terminus des GPCR, um ein GPCR-Fusionspartner-Protein zu bilden, wobei das Fusionspartner-Protein eine Aminosäuresequenz umfasst, die zu mindestens 90 % mit der Aminosäuresequenz des Rubredoxins homolog ist,
b) Exprimieren und Aufreinigen des GPCR-Fusionspartner-Proteins und
c) Kristallisieren des aufgereinigten GPCR-Fusionspartner-Proteins
umfasst.

9. Das Verfahren nach den Ansprüchen 7 und 8, wobei das GPCR ein GPCR der Klasse A ist.

10. Das Verfahren nach den Ansprüchen 7 bis 9, wobei das Verfahren ferner die Stufe des Kristallisierens des aufgereinigten GPCR-Fusionspartner-Proteins, das in Gegenwart eines Kristallisationshilfsmittels durchgeführt wird, umfasst.

## Revendications

1. Composition comprenant une protéine de fusion GPCR, qui comprend de l'extrémité N à l'extrémité C : (i) un premier domaine comprenant une partie de récepteur couplé à la protéine G (GPCR), où le premier domaine comprend les premier à cinquième domaines transmembranaires de GPCR, (ii) un deuxième domaine comprenant une séquence d'acides aminés partenaire de fusion, ladite séquence étant homologue à au moins 90% à la rubrédoxine, et (iii) un troisième domaine comprenant une partie du GPCR où le troisième domaine comprend les sixième et septième domaines transmembranaires de GPCR.

2. Composition comprenant une protéine de fusion GPCR, qui comprend de l'extrémité N à l'extrémité C : (i) un premier domaine comprenant un récepteur couplé à la protéine G (GPCR), et (ii) un deuxième domaine comprenant une séquence d'acides aminés partenaire de fusion, ladite séquence étant homologue à au moins 90% à la rubrédoxine.

3. Composition comprenant une protéine de fusion GPCR, qui comprend de l'extrémité N à l'extrémité C : (i) un premier domaine comprenant une séquence d'acides aminés partenaire de fusion, ladite séquence étant homologue à au moins 90% à la rubrédoxine, et (ii) un deuxième domaine comprenant un GPCR.

4. Composition selon les revendications 1 à 3, où la protéine de fusion GPCR est sous forme cristalline.

5. Composition selon la revendication 4, où la protéine de fusion GPCR est de qualité suffisante pour réaliser une détermination de structure cristallographique par rayons X pour le GPCR à une résolution d'au moins 3 angströms.

6. Composition selon la revendication 1, où le partenaire de fusion comprend le domaine correspondant au troisième domaine intracellulaire de GPCR entre le cinquième et le sixième domaine transmembranaire de GPCR.

7. Procédé d'utilisation d'un partenaire de fusion pour supporter la cristallisation d'une protéine pour des études structurales cristallographiques d'un récepteur couplé à la protéine G (GPCR), comprenant :
(a) l'incorporation d'un partenaire de fusion dans un domaine intracellulaire de GPCR pour former une protéine de fusion GPCR, où le partenaire de fusion comprend une séquence d'acides aminés qui est homologue à au moins 90% à la rubrédoxine ;
(b) l'expression et la purification de la protéine de fusion GPCR, et
(c) la cristallisation de la protéine de fusion GPCR purifiée.

8. Procédé d'utilisation d'un partenaire de fusion pour supporter la cristallisation d'une protéine pour des études structurales cristallographiques d'un récepteur couplé à la protéine G (GPCR), comprenant :
(a) l'attachement d'un partenaire de fusion à l'extrémité N ou l'extrémité C de GPCR pour former une protéine de fusion GPCR, où le partenaire de fusion comprend une séquence d'acides aminés qui est homologue à au moins 90% à la rubrédoxine ;
(b) l'expression et la purification de la protéine de fusion GPCR, et
(c) la cristallisation de la protéine de fusion GPCR purifiée.

9. Procédé selon les revendications 7 et 8, où le GPCR est un GPCR de classe A.

10. Procédé selon les revendications 7 à 9, où le procédé comprend en outre, le fait que l'étape de cristallisation de la protéine de fusion GPCR purifiée est réalisée en présence d'un additif de cristallisation.


### SEQUENCE LISTING

<110> Receptos, Inc. The Scripps Research Institute HANSON, Michael A. ROTH, Christopher B. STEVENS, Raymond C. KUNKEN, Joshua M. GRIFFITH, Mark T. THOMPSON, Aaron A. LIU, Wei XU, Fei KATRITCH, Vsevolod
<120> NOVEL FUSION PARTNERS FOR THE PURPOSE OF CRYSTALLIZING G-PROTEIN COUPLED RECEPTORS
<130> 018410-0406491
<140> To Be Assigned
   <141> 2012-05-10
<150> 61/485,872
   <151> 2011-05-13
<160> 13
<170> PatentIn version 3.5
<210> 1
   <211> 381
   <212> PRT
   <213> Homo sapiens
<400> 1
   Met Gly Pro Thr Ser Val Pro Leu Val Lys Ala His Arg Ser Ser Val
   1               5                   10                  15
   Ser Asp Tyr Val Asn Tyr Asp Ile Ile Val Arg His Tyr Asn Tyr Thr
               20                  25                  30
   Gly Lys Leu Asn Ile Ser Ala Asp Lys Glu Asn Ser Ile Lys Leu Thr
           35                  40                  45
   Ser Val Val Phe Ile Leu Ile Cys Cys Phe Ile Ile Leu Glu Asn Ile
       50                  55                  60
   Phe Val Leu Leu Thr Ile Trp Lys Thr Lys Lys Phe His Arg Pro Met
   65                  70                  75                  80
   Tyr Tyr Phe Ile Gly Asn Leu Ala Leu Ser Asp Leu Leu Ala Gly Val
                   85                  90                  95
   Ala Tyr Thr Ala Asn Leu Leu Leu Ser Gly Ala Thr Thr Tyr Lys Leu
               100                 105                 110
   Thr Pro Ala Gln Trp Phe Leu Arg Glu Gly Ser Met Phe Val Ala Leu
           115                 120                 125
   Ser Ala Ser Val Phe Ser Leu Leu Ala Ile Ala Ile Glu Arg Tyr Ile
       130                 135                 140
   Thr Met Leu Lys Met Lys Leu His Asn Gly Ser Asn Asn Phe Arg Leu
   145                 150                 155                 160
   Phe Leu Leu Ile Ser Ala Cys Trp Val Ile Ser Leu Ile Leu Gly Gly
                   165                 170                 175
   Leu Pro Ile Met Gly Trp Asn Cys Ile Ser Ala Leu Ser Ser Cys Ser
               180                 185                 190
   Thr Val Leu Pro Leu Tyr His Lys His Tyr Ile Leu Phe Cys Thr Thr
           195                 200                 205
   Val Phe Thr Leu Leu Leu Leu Ser Ile Val Ile Leu Tyr Cys Arg Ile
       210                 215                 220
   Tyr Ser Leu Val Arg Thr Arg Ser Arg Arg Leu Thr Phe Arg Lys Asn
   225                 230                 235                 240
   Ile Ser Lys Ala Ser Arg Ser Ser Glu Asn Val Ala Leu Leu Lys Thr
                   245                 250                 255
   Val Ile Ile Val Leu Ser Val Phe Ile Ala Cys Trp Ala Pro Leu Phe
               260                 265                 270
   Ile Leu Leu Leu Leu Asp Val Gly Cys Lys Val Lys Thr Cys Asp Ile
           275                 280                 285
   Leu Phe Arg Ala Glu Tyr Phe Leu Val Leu Ala Val Leu Asn Ser Gly
       290                 295                 300
   Thr Asn Pro Ile Ile Tyr Thr Leu Thr Asn Lys Glu Met Arg Arg Ala
   305                 310                 315                 320
   Phe Ile Arg Ile Met Ser Cys Cys Lys Cys Pro Ser Gly Asp Ser Ala
                   325                 330                 335
   Gly Lys Phe Lys Arg Pro Ile Ile Ala Gly Met Glu Phe Ser Arg Ser
               340                 345                 350
   Lys Ser Asp Asn Ser Ser His Pro Gln Lys Asp Glu Gly Asp Asn Pro
           355                 360                 365
   Glu Thr Ile Met Ser Ser Gly Asn Val Asn Ser Ser Ser
       370                 375                 380
<210> 2
   <211> 419
   <212> PRT
   <213> Homo sapiens
<400> 2
   Met Gly Pro Thr Ser Val Pro Leu Val Lys Ala His Arg Ser Ser Val
   1               5                   10                  15
   Ser Asp Tyr Val Asn Tyr Asp Ile Ile Val Arg His Tyr Asn Tyr Thr
               20                  25                  30
   Gly Lys Leu Asn Ile Ser Ala Asp Lys Glu Asn Ser Ile Lys Leu Thr
           35                  40                  45
   Ser Val Val Phe Ile Leu Ile Cys Cys Phe Ile Ile Leu Glu Asn Ile
       50                  55                  60
   Phe Val Leu Leu Thr Ile Trp Lys Thr Lys Lys Phe His Arg Pro Met
   65                  70                  75                  80
   Tyr Tyr Phe Ile Gly Asn Leu Ala Leu Ser Asp Leu Leu Ala Gly Val
                   85                  90                  95
   Ala Tyr Thr Ala Asn Leu Leu Leu Ser Gly Ala Thr Thr Tyr Lys Leu
               100                 105                 110
   Thr Pro Ala Gln Trp Phe Leu Arg Glu Gly Ser Met Phe Val Ala Leu
           115                 120                 125
   Ser Ala Ser Val Phe Ser Leu Leu Ala Ile Ala Ile Glu Arg Tyr Ile
       130                 135                 140
   Thr Met Leu Lys Met Lys Leu His Asn Gly Ser Asn Asn Phe Arg Leu
   145                 150                 155                 160
   Phe Leu Leu Ile Ser Ala Cys Trp Val Ile Ser Leu Ile Leu Gly Gly
                   165                 170                 175
   Leu Pro Ile Met Gly Trp Asn Cys Ile Ser Ala Leu Ser Ser Cys Ser
               180                 185                 190
   Thr Val Leu Pro Leu Tyr His Lys His Tyr Ile Leu Phe Cys Thr Thr
           195                 200                 205
   Val Phe Thr Leu Leu Leu Leu Ser Ile Val Ile Leu Tyr Cys Arg Ile
   210 215 220
   Tyr Ser Leu Val Arg Thr Arg Ala Asp Leu Glu Asp Asn Trp Glu Thr
   225 230 235 240
   Leu Asn Asp Asn Leu Lys Val Ile Glu Lys Ala Asp Asn Ala Ala Gln
   245 250 255
   Val Lys Asp Ala Leu Thr Lys Met Arg Ala Ala Ala Leu Asp Ala Gln
   260 265 270
   Lys Ala Thr Pro Pro Lys Leu Glu Asp Lys Ser Pro Asp Ser Pro Glu
   275 280 285
   Met Lys Asp Phe Arg His Gly Phe Asp Ile Leu Val Gly Gln Ile Asp
   290 295 300
   Asp Ala Leu Lys Leu Ala Asn Glu Gly Lys Val Lys Glu Ala Gln Ala
   305 310 315 320
   Ala Ala Glu Gln Leu Lys Thr Thr Arg Asn Ala Tyr Ile Gln Lys Tyr
   325 330 335
   Leu Ala Ser Arg Ser Ser Glu Asn Val Ala Leu Leu Lys Thr Val Ile
   340 345 350
   Ile Val Leu Ser Val Phe Ile Ala Cys Trp Ala Pro Leu Phe Ile Leu
   355 360 365
   Leu Leu Leu Asp Val Gly Cys Lys Val Lys Thr Cys Asp Ile Leu Phe
   370 375 380
   Arg Ala Glu Tyr Phe Leu Val Leu Ala Val Leu Asn Ser Gly Thr Asn
   385 390 395 400
   Pro Ile Ile Tyr Thr Leu Thr Asn Lys Glu Met Arg Arg Ala Phe Ile
   405 410 415
   Arg Ile Met
<210> 3
   <211> 460
   <212> PRT
   <213> Homo sapiens
<400> 3
   Met Gly Pro Thr Ser Val Pro Leu Val Lys Ala His Arg Ser Ser Val
   1               5                   10                  15
   Ser Asp Tyr Val Asn Tyr Asp Ile Ile Val Arg His Tyr Asn Tyr Thr
               20                  25                  30
   Gly Lys Leu Asn Ile Ser Ala Asp Lys Glu Asn Ser Ile Lys Leu Thr
           35                  40                  45
   Ser Val Val Phe Ile Leu Ile Cys Cys Phe Ile Ile Leu Glu Asn Ile
       50                  55                  60
   Phe Val Leu Leu Thr Ile Trp Lys Thr Lys Lys Phe His Arg Pro Met
   65                  70                  75                  80
   Tyr Tyr Phe Ile Gly Asn Leu Ala Leu Ser Asp Leu Leu Ala Gly Val
                   85                  90                  95
   Ala Tyr Thr Ala Asn Leu Leu Leu Ser Gly Ala Thr Thr Tyr Lys Leu
               100                 105                 110
   Thr Pro Ala Gln Trp Phe Leu Arg Glu Gly Ser Met Phe Val Ala Leu
           115                 120                 125
   Ser Ala Ser Val Phe Ser Leu Leu Ala Ile Ala Ile Glu Arg Tyr Ile
       130                 135                 140
   Thr Met Leu Lys Met Lys Leu His Asn Gly Ser Asn Asn Phe Arg Leu
   145                 150                 155                 160
   Phe Leu Leu Ile Ser Ala Cys Trp Val Ile Ser Leu Ile Leu Gly Gly
                   165                 170                 175
   Leu Pro Ile Met Gly Trp Asn Cys Ile Ser Ala Leu Ser Ser Cys Ser
               180                 185                 190
   Thr Val Leu Pro Leu Tyr His Lys His Tyr Ile Leu Phe Cys Thr Thr
           195                 200                 205
   Val Phe Thr Leu Leu Leu Leu Ser Ile Val Ile Leu Tyr Cys Arg Ile
       210                 215                 220
   Tyr Ser Leu Val Arg Thr Arg Ala Lys Ala Leu Ile Val Tyr Gly Ser
   225                 230                 235                 240
   Thr Thr Gly Asn Thr Glu Tyr Thr Ala Glu Thr Ile Ala Arg Glu Leu
                   245                 250                 255
   Ala Asp Ala Gly Tyr Glu Val Asp Ser Arg Asp Ala Ala Ser Val Glu
               260                 265                 270
   Ala Gly Gly Leu Phe Glu Gly Phe Asp Leu Val Leu Leu Gly Cys Ser
           275                 280                 285
   Thr Trp Gly Asp Asp Ser Ile Glu Leu Gln Asp Asp Phe Ile Pro Leu
       290                 295                 300
   Phe Asp Ser Leu Glu Glu Thr Gly Ala Gln Gly Arg Lys Val Ala Cys
   305                 310                 315                 320
   Phe Gly Cys Gly Asp Ser Ser Trp Glu Tyr Phe Cys Gly Ala Val Asp
                   325                 330                 335
   Ala Ile Glu Glu Lys Leu Lys Asn Leu Gly Ala Glu Ile Val Gln Asp
               340                 345                 350
   Gly Leu Arg Ile Asp Gly Asp Pro Arg Ala Ala Arg Asp Asp Ile Val
           355                 360                 365
   Gly Trp Ala His Asp Val Arg Gly Ala Ile Ala Ser Arg Ser Ser Glu
       370                 375                 380
   Asn Val Ala Leu Leu Lys Thr Val Ile Ile Val Leu Ser Val Phe Ile
   385                 390                 395                 400
   Ala Cys Trp Ala Pro Leu Phe Ile Leu Leu Leu Leu Asp Val Gly Cys
                   405                 410                 415
   Lys Val Lys Thr Cys Asp Ile Leu Phe Arg Ala Glu Tyr Phe Leu Val
               420                 425                 430
   Leu Ala Val Leu Asn Ser Gly Thr Asn Pro Ile Ile Tyr Thr Leu Thr
           435                 440                 445
   Asn Lys Glu Met Arg Arg Ala Phe Ile Arg Ile Met
       450                 455                 460
<210> 4
   <211> 498
   <212> PRT
   <213> Homo sapiens
<400> 4
   Met Gly Pro Thr Ser Val Pro Leu Val Lys Ala His Arg Ser Ser Val
   1               5                   10                  15
   Ser Asp Tyr Val Asn Tyr Asp Ile Ile Val Arg His Tyr Asn Tyr Thr
               20                  25                  30
   Gly Lys Leu Asn Ile Ser Ala Asp Lys Glu Asn Ser Ile Lys Leu Thr
           35                  40                  45
   Ser Val Val Phe Ile Leu Ile Cys Cys Phe Ile Ile Leu Glu Asn Ile
       50                  55                  60
   Phe Val Leu Leu Thr Ile Trp Lys Thr Lys Lys Phe His Arg Pro Met
   65                  70                  75                  80
   Tyr Tyr Phe Ile Gly Asn Leu Ala Leu Ser Asp Leu Leu Ala Gly Val
                   85                  90                  95
   Ala Tyr Thr Ala Asn Leu Leu Leu Ser Gly Ala Thr Thr Tyr Lys Leu
               100                 105                 110
   Thr Pro Ala Gln Trp Phe Leu Arg Glu Gly Ser Met Phe Val Ala Leu
           115                 120                 125
   Ser Ala Ser Val Phe Ser Leu Leu Ala Ile Ala Ile Glu Arg Tyr Ile
       130                 135                 140
   Thr Met Leu Lys Met Lys Leu His Asn Gly Ser Asn Asn Phe Arg Leu
   145                 150                 155                 160
   Phe Leu Leu Ile Ser Ala Cys Trp Val Ile Ser Leu Ile Leu Gly Gly
                   165                 170                 175
   Leu Pro Ile Met Gly Trp Asn Cys Ile Ser Ala Leu Ser Ser Cys Ser
               180                 185                 190
   Thr Val Leu Pro Leu Tyr His Lys His Tyr Ile Leu Phe Cys Thr Thr
           195                 200                 205
   Val Phe Thr Leu Leu Leu Leu Ser Ile Val Ile Leu Tyr Cys Arg Ile
       210                 215                 220
   Tyr Ser Leu Val Arg Thr Arg Ala Ser Thr Asp Tyr Trp Gln Asn Trp
   225                 230                 235                 240
   Thr Phe Gly Gly Gly Ile Val Asn Ala Val Asn Gly Ser Gly Gly Asn
                   245                 250                 255
   Tyr Ser Val Asn Trp Ser Asn Thr Gly Asn Phe Val Val Gly Lys Gly
               260                 265                 270
   Trp Thr Thr Gly Ser Pro Phe Arg Thr Ile Asn Tyr Asn Ala Gly Val
           275                 280                 285
   Trp Ala Pro Asn Gly Asn Gly Tyr Leu Thr Leu Tyr Gly Trp Thr Arg
       290                 295                 300
   Ser Pro Leu Ile Glu Tyr Tyr Val Val Asp Ser Trp Gly Thr Tyr Arg
   305                 310                 315                 320
   Pro Thr Gly Thr Tyr Lys Gly Thr Val Lys Ser Asp Gly Gly Thr Tyr
                   325                 330                 335
   Asp Ile Tyr Thr Thr Thr Arg Tyr Asn Ala Pro Ser Ile Asp Gly Asp
               340                 345                 350
   Asp Thr Thr Phe Thr Gln Tyr Trp Ser Val Arg Gln Ser Lys Arg Pro
           355                 360                 365
   Thr Gly Ser Asn Ala Thr Ile Thr Phe Thr Asn His Val Asn Ala Trp
       370                 375                 380
   Lys Ser His Gly Met Asn Leu Gly Ser Asn Trp Ala Tyr Gln Val Met
   385                 390                 395                 400
   Ala Thr Glu Gly Tyr Gln Ser Ser Gly Ser Ser Asn Val Thr Val Trp
                   405                 410                 415
   Ala Ser Arg Ser Ser Glu Asn Val Ala Leu Leu Lys Thr Val Ile Ile
               420                 425                 430
   Val Leu Ser Val Phe Ile Ala Cys Trp Ala Pro Leu Phe Ile Leu Leu
           435                 440                 445
   Leu Leu Asp Val Gly Cys Lys Val Lys Thr Cys Asp Ile Leu Phe Arg
       450                 455                 460
   Ala Glu Tyr Phe Leu Val Leu Ala Val Leu Asn Ser Gly Thr Asn Pro
   465                 470                 475                 480
   Ile Ile Tyr Thr Leu Thr Asn Lys Glu Met Arg Arg Ala Phe Ile Arg
                   485                 490                 495
   Ile Met
<210> 5
   <211> 367
   <212> PRT
   <213> Homo sapiens
<400> 5
   Met Gly Pro Thr Ser Val Pro Leu Val Lys Ala His Arg Ser Ser Val
   1               5                   10                  15
   Ser Asp Tyr Val Asn Tyr Asp Ile Ile Val Arg His Tyr Asn Tyr Thr
               20                  25                  30
   Gly Lys Leu Asn Ile Ser Ala Asp Lys Glu Asn Ser Ile Lys Leu Thr
           35                  40                  45
   Ser Val Val Phe Ile Leu Ile Cys Cys Phe Ile Ile Leu Glu Asn Ile
       50                  55                  60
   Phe Val Leu Leu Thr Ile Trp Lys Thr Lys Lys Phe His Arg Pro Met
   65                  70                  75                  80
   Tyr Tyr Phe Ile Gly Asn Leu Ala Leu Ser Asp Leu Leu Ala Gly Val
                   85                  90                  95
   Ala Tyr Thr Ala Asn Leu Leu Leu Ser Gly Ala Thr Thr Tyr Lys Leu
               100                 105                 110
   Thr Pro Ala Gln Trp Phe Leu Arg Glu Gly Ser Met Phe Val Ala Leu
           115                 120                 125
   Ser Ala Ser Val Phe Ser Leu Leu Ala Ile Ala Ile Glu Arg Tyr Ile
       130                 135                 140
   Thr Met Leu Lys Met Lys Leu His Asn Gly Ser Asn Asn Phe Arg Leu
   145                 150                 155                 160
   Phe Leu Leu Ile Ser Ala Cys Trp Val Ile Ser Leu Ile Leu Gly Gly
                   165                 170                 175
   Leu Pro Ile Met Gly Trp Asn Cys Ile Ser Ala Leu Ser Ser Cys Ser
               180                 185                 190
   Thr Val Leu Pro Leu Tyr His Lys His Tyr Ile Leu Phe Cys Thr Thr
           195                 200                 205
   Val Phe Thr Leu Leu Leu Leu Ser Ile Val Ile Leu Tyr Cys Arg Ile
       210                 215                 220
   Tyr Ser Leu Val Arg Thr Arg Met Lys Lys Tyr Thr Cys Thr Val Cys
   225                 230                 235                 240
   Gly Tyr Ile Tyr Asn Pro Glu Asp Gly Asp Pro Asp Asn Gly Val Asn
                   245                 250                 255
   Pro Gly Thr Asp Phe Lys Asp Ile Pro Asp Asp Trp Val Cys Pro Leu
               260                 265                 270
   Cys Gly Val Gly Lys Asp Gln Phe Glu Glu Val Glu Glu Ala Ser Arg
           275                 280                 285
   Ser Ser Glu Asn Val Ala Leu Leu Lys Thr Val Ile Ile Val Leu Ser
       290                 295                 300
   Val Phe Ile Ala Cys Trp Ala Pro Leu Phe Ile Leu Leu Leu Leu Asp
   305                 310                 315                 320
   Val Gly Cys Lys Val Lys Thr Cys Asp Ile Leu Phe Arg Ala Glu Tyr
                   325                 330                 335
   Phe Leu Val Leu Ala Val Leu Asn Ser Gly Thr Asn Pro Ile Ile Tyr
               340                 345                 350
   Thr Leu Thr Asn Lys Glu Met Arg Arg Ala Phe Ile Arg Ile Met
           355                 360                 365
<210> 6
   <211> 466
   <212> PRT
   <213> Homo sapiens
<400> 6
   Met Lys Thr Ile Ile Ala Leu Ser Tyr Ile Phe Cys Leu Val Phe Ala
   1               5                   10                  15
   Gly Ala Pro Gly Pro Thr Ser Val Pro Leu Val Lys Ala His Arg Ser
               20                  25                  30
   Ser Val Ser Asp Tyr Val Asn Tyr Asp Ile Ile Val Arg His Tyr Asn
           35                  40                  45
   Tyr Thr Gly Lys Leu Asn Ile Ser Ala Asp Lys Glu Asn Ser Ile Lys
       50                  55                  60
   Leu Thr Ser Val Val Phe Ile Leu Ile Cys Cys Phe Ile Ile Leu Glu
   65                  70                  75                  80
   Asn Ile Phe Val Leu Leu Thr Ile Trp Lys Thr Lys Lys Phe His Arg
                   85                  90                  95
   Pro Met Tyr Tyr Phe Ile Gly Asn Leu Ala Leu Ser Asp Leu Leu Ala
               100                 105                 110
   Gly Val Ala Tyr Thr Ala Asn Leu Leu Leu Ser Gly Ala Thr Thr Tyr
           115                 120                 125
   Lys Leu Thr Pro Ala Gln Trp Phe Leu Arg Glu Gly Ser Met Phe Val
       130                 135                 140
   Ala Leu Ser Ala Ser Val Phe Ser Leu Leu Ala Ile Ala Ile Glu Arg
   145                 150                 155                 160
   Tyr Ile Thr Met Leu Lys Met Lys Leu His Asn Gly Ser Asn Asn Phe
                   165                 170                 175
   Arg Leu Phe Leu Leu Ile Ser Ala Cys Trp Val Ile Ser Leu Ile Leu
               180                 185                 190
   Gly Gly Leu Pro Ile Met Gly Trp Asn Cys Ile Ser Ala Leu Ser Ser
           195                 200                 205
   Cys Ser Thr Val Leu Pro Leu Tyr His Lys His Tyr Ile Leu Phe Cys
       210                 215                 220
   Thr Thr Val Phe Thr Leu Leu Leu Leu Ser Ile Val Ile Leu Tyr Cys
   225                 230                 235                 240
   Arg Ile Tyr Ser Leu Val Arg Thr Arg Ala Asp Leu Glu Asp Asn Trp
                   245                 250                 255
   Glu Thr Leu Asn Asp Asn Leu Lys Val Ile Glu Lys Ala Asp Asn Ala
               260                 265                 270
   Ala Gln Val Lys Asp Ala Leu Thr Lys Met Arg Ala Ala Ala Leu Asp
           275                 280                 285
   Ala Gln Lys Ala Thr Pro Pro Lys Leu Glu Asp Lys Ser Pro Asp Ser
       290                 295                 300
   Pro Glu Met Lys Asp Phe Arg His Gly Phe Asp Ile Leu Val Gly Gln
   305                 310                 315                 320
   Ile Asp Asp Ala Leu Lys Leu Ala Asn Glu Gly Lys Val Lys Glu Ala
                   325                 330                 335
   Gln Ala Ala Ala Glu Gln Leu Lys Thr Thr Arg Asn Ala Tyr Ile Gln
   340 345 350
   Lys Tyr Leu Ala Ser Arg Ser Ser Glu Asn Val Ala Leu Leu Lys Thr
   355 360 365
   Val Ile Ile Val Leu Ser Val Phe Ile Ala Cys Trp Ala Pro Leu Phe
   370 375 380
   Ile Leu Leu Leu Leu Asp Val Gly Cys Lys Val Lys Thr Cys Asp Ile
   385 390 395 400
   Leu Phe Arg Ala Glu Tyr Phe Leu Val Leu Ala Val Leu Asn Ser Gly
   405 410 415
   Thr Asn Pro Ile Ile Tyr Thr Leu Thr Asn Lys Glu Met Arg Arg Ala
   420 425 430
   Phe Ile Arg Ile Met Gly Arg Pro Leu Glu Val Leu Phe Gln Gly Pro
   435 440 445
   His His His His His His His His His His Asp Tyr Lys Asp Asp Asp
   450 455 460
   Asp Lys
   465
<210> 7
   <211> 254
   <212> PRT
   <213> Homo sapiens
<400> 7
   Met Lys Thr Ile Ile Ala Leu Ser Tyr Ile Phe Cys Leu Val Phe Ala
   1 5 10 15
   Asp Tyr Lys Asp Asp Asp Asp Ala Met Gly Gln Pro Gly Asn Gly Ser
   20 25 30
   Ala Phe Leu Leu Ala Pro Asn Arg Ser His Ala Pro Asp His Asp Val
   35 40 45
   Thr Gln Gln Arg Asp Glu Val Trp Val Val Gly Met Gly Ile Val Met
   50 55 60
   Ser Leu Ile Val Leu Ala Ile Val Phe Gly Asn Val Leu Val Ile Thr
   65 70 75 80
   Ala Ile Ala Lys Phe Glu Arg Leu Gln Thr Val Thr Asn Tyr Phe Ile
                   85                  90                  95
   Thr Ser Leu Ala Cys Ala Asp Leu Val Met Gly Leu Ala Val Val Pro
               100                 105                 110
   Phe Gly Ala Ala His Ile Leu Met Lys Met Trp Thr Phe Gly Asn Phe
           115                 120                 125
   Trp Cys Glu Phe Trp Thr Ser Ile Asp Val Leu Cys Val Thr Ala Ser
       130                 135                 140
   Ile Trp Thr Leu Cys Val Ile Ala Val Asp Arg Tyr Phe Ala Ile Thr
   145                 150                 155                 160
   Ser Pro Phe Lys Tyr Gln Ser Leu Leu Thr Lys Asn Lys Ala Arg Val
                   165                 170                 175
   Ile Ile Leu Met Val Trp Ile Val Ser Gly Leu Thr Ser Phe Leu Pro
               180                 185                 190
   Ile Gln Met His Trp Tyr Arg Ala Thr His Gln Glu Ala Ile Asn Cys
           195                 200                 205
   Tyr Ala Glu Glu Thr Cys Cys Asp Phe Phe Thr Asn Gln Ala Tyr Ala
       210                 215                 220
   Ile Ala Ser Ser Ile Val Ser Phe Tyr Val Pro Leu Val Ile Met Val
   225                 230                 235                 240
   Phe Val Tyr Ser Arg Val Phe Gln Glu Ala Lys Arg Gln Leu
                   245                 250
<210> 8
   <211> 506
   <212> PRT
   <213> Homo sapiens
<400> 8
   Met Lys Thr Ile Ile Ala Leu Ser Tyr Ile Phe Cys Leu Val Phe Ala
   1               5                   10                  15
   Asp Tyr Lys Asp Asp Asp Asp Ala Met Gly Gln Pro Gly Asn Gly Ser
               20                  25                  30
   Ala Phe Leu Leu Ala Pro Asn Arg Ser His Ala Pro Asp His Asp Val
           35                  40                  45
   Thr Gln Gln Arg Asp Glu Val Trp Val Val Gly Met Gly Ile Val Met
       50                  55                  60
   Ser Leu Ile Val Leu Ala Ile Val Phe Gly Asn Val Leu Val Ile Thr
   65                  70                  75                  80
   Ala Ile Ala Lys Phe Glu Arg Leu Gln Thr Val Thr Asn Tyr Phe Ile
                   85                  90                  95
   Thr Ser Leu Ala Cys Ala Asp Leu Val Met Gly Leu Ala Val Val Pro
               100                 105                 110
   Phe Gly Ala Ala His Ile Leu Met Lys Met Trp Thr Phe Gly Asn Phe
           115                 120                 125
   Trp Cys Glu Phe Trp Thr Ser Ile Asp Val Leu Cys Val Thr Ala Ser
       130                 135                 140
   Ile Trp Thr Leu Cys Val Ile Ala Val Asp Arg Tyr Phe Ala Ile Thr
   145                 150                 155                 160
   Ser Pro Phe Lys Tyr Gln Ser Leu Leu Thr Lys Asn Lys Ala Arg Val
                   165                 170                 175
   Ile Ile Leu Met Val Trp Ile Val Ser Gly Leu Thr Ser Phe Leu Pro
               180                 185                 190
   Ile Gln Met His Trp Tyr Arg Ala Thr His Gln Glu Ala Ile Asn Cys
           195                 200                 205
   Tyr Ala Glu Glu Thr Cys Cys Asp Phe Phe Thr Asn Gln Ala Tyr Ala
       210                 215                 220
   Ile Ala Ser Ser Ile Val Ser Phe Tyr Val Pro Leu Val Ile Met Val
   225                 230                 235                 240
   Phe Val Tyr Ser Arg Val Phe Gln Glu Ala Lys Arg Gln Leu Asn Ile
                   245                 250                 255
   Phe Glu Met Leu Arg Ile Asp Glu Gly Leu Arg Leu Lys Ile Tyr Lys
               260                 265                 270
   Asp Thr Glu Gly Tyr Tyr Thr Ile Gly Ile Gly His Leu Leu Thr Lys
           275                 280                 285
   Ser Pro Ser Leu Asn Ala Ala Lys Ser Glu Leu Asp Lys Ala Ile Gly
   290 295 300
   Arg Asn Thr Asn Gly Val Ile Thr Lys Asp Glu Ala Glu Lys Leu Phe
   305 310 315 320
   Asn Gln Asp Val Asp Ala Ala Val Arg Gly Ile Leu Arg Asn Ala Lys
   325 330 335
   Leu Lys Pro Val Tyr Asp Ser Leu Asp Ala Val Arg Arg Ala Ala Leu
   340 345 350
   Ile Asn Met Val Phe Gln Met Gly Glu Thr Gly Val Ala Gly Phe Thr
   355 360 365
   Asn Ser Leu Arg Met Leu Gln Gln Lys Arg Trp Asp Glu Ala Ala Val
   370 375 380
   Asn Leu Ala Lys Ser Arg Trp Tyr Asn Gln Thr Pro Asn Arg Ala Lys
   385 390 395 400
   Arg Val Ile Thr Thr Phe Arg Thr Gly Thr Trp Asp Ala Tyr Lys Phe
   405 410 415
   Cys Leu Lys Glu His Lys Ala Leu Lys Thr Leu Gly Ile Ile Met Gly
   420 425 430
   Thr Phe Thr Leu Cys Trp Leu Pro Phe Phe Ile Val Asn Ile Val His
   435 440 445
   Val Ile Gln Asp Asn Leu Ile Arg Lys Glu Val Tyr Ile Leu Leu Asn
   450 455 460
   Trp Ile Gly Tyr Val Asn Ser Gly Phe Asn Pro Leu Ile Tyr Cys Arg
   465 470 475 480
   Ser Pro Asp Phe Arg Ile Ala Phe Gln Glu Leu Leu Cys Leu Arg Arg
   485 490 495
   Ser Ser Leu Lys His His His His His His
   500 505
<210> 9
   <211> 473
   <212> PRT
   <213> Homo sapiens
<400> 9
   Met Lys Thr Ile Ile Ala Leu Ser Tyr Ile Phe Cys Leu Val Phe Ala
   1               5                   10                  15
   Asp Tyr Lys Asp Asp Asp Asp Ala Met Gly Gln Pro Gly Asn Gly Ser
               20                  25                  30
   Ala Phe Leu Leu Ala Pro Asn Arg Ser His Ala Pro Asp His Asp Val
           35                  40                  45
   Thr Gln Gln Arg Asp Glu Val Trp Val Val Gly Met Gly Ile Val Met
       50                  55                  60
   Ser Leu Ile Val Leu Ala Ile Val Phe Gly Asn Val Leu Val Ile Thr
   65                  70                  75                  80
   Ala Ile Ala Lys Phe Glu Arg Leu Gln Thr Val Thr Asn Tyr Phe Ile
                   85                  90                  95
   Thr Ser Leu Ala Cys Ala Asp Leu Val Met Gly Leu Ala Val Val Pro
               100                 105                 110
   Phe Gly Ala Ala His Ile Leu Met Lys Met Trp Thr Phe Gly Asn Phe
           115                 120                 125
   Trp Cys Glu Phe Trp Thr Ser Ile Asp Val Leu Cys Val Thr Ala Ser
       130                 135                 140
   Ile Trp Thr Leu Cys Val Ile Ala Val Asp Arg Tyr Phe Ala Ile Thr
   145                 150                 155                 160
   Ser Pro Phe Lys Tyr Gln Ser Leu Leu Thr Lys Asn Lys Ala Arg Val
                   165                 170                 175
   Ile Ile Leu Met Val Trp Ile Val Ser Gly Leu Thr Ser Phe Leu Pro
               180                 185                 190
   Ile Gln Met His Trp Tyr Arg Ala Thr His Gln Glu Ala Ile Asn Cys
           195                 200                 205
   Tyr Ala Glu Glu Thr Cys Cys Asp Phe Phe Thr Asn Gln Ala Tyr Ala
       210                 215                 220
   Ile Ala Ser Ser Ile Val Ser Phe Tyr Val Pro Leu Val Ile Met Val
   225                 230                 235                 240
   Phe Val Tyr Ser Arg Val Phe Gln Glu Ala Lys Arg Gln Leu Lys Asp
                   245                 250                 255
   Glu Ala Glu Lys Leu Phe Asn Gln Asp Val Asp Ala Ala Val Arg Gly
               260                 265                 270
   Ile Leu Arg Asn Ala Lys Leu Lys Pro Val Tyr Asp Ser Leu Asp Ala
           275                 280                 285
   Val Arg Arg Ala Ala Leu Ile Asn Met Val Phe Gln Met Gly Glu Thr
       290                 295                 300
   Gly Val Ala Gly Phe Thr Asn Ser Leu Arg Met Leu Gln Gln Lys Arg
   305                 310                 315                 320
   Trp Asp Glu Ala Ala Val Asn Leu Ala Lys Ser Arg Trp Tyr Asn Gln
                   325                 330                 335
   Thr Pro Asn Arg Ala Lys Arg Val Ile Thr Thr Phe Arg Thr Gly Thr
               340                 345                 350
   Trp Asp Ala Tyr Lys Asn Leu Ser Gly Gly Gly Gly Ala Met Asp Ile
           355                 360                 365
   Phe Glu Met Leu Arg Ile Asp Glu Gly Lys Phe Cys Leu Lys Glu His
       370                 375                 380
   Lys Ala Leu Lys Thr Leu Gly Ile Ile Met Gly Thr Phe Thr Leu Cys
   385                 390                 395                 400
   Trp Leu Pro Phe Phe Ile Val Asn Ile Val His Val Ile Gln Asp Asn
                   405                 410                 415
   Leu Ile Arg Lys Glu Val Tyr Ile Leu Leu Asn Trp Ile Gly Tyr Val
               420                 425                 430
   Asn Ser Gly Phe Asn Pro Leu Ile Tyr Cys Arg Ser Pro Asp Phe Arg
           435                 440                 445
   Ile Ala Phe Gln Glu Leu Leu Cys Leu Arg Arg Ser Ser Leu Lys His
       450                 455                 460
   His His His His His His His His His
   465                 470
<210> 10
   <211> 455
   <212> PRT
   <213> Homo sapiens
<400> 10
   Met Lys Thr Ile Ile Ala Leu Ser Tyr Ile Phe Cys Leu Val Phe Ala
   1               5                   10                  15
   Asp Tyr Lys Asp Asp Asp Asp Ala Met Gly Gln Pro Gly Asn Gly Ser
               20                  25                  30
   Ala Phe Leu Leu Ala Pro Asn Arg Ser His Ala Pro Asp His Asp Val
           35                  40                  45
   Thr Gln Gln Arg Asp Glu Val Trp Val Val Gly Met Gly Ile Val Met
       50                  55                  60
   Ser Leu Ile Val Leu Ala Ile Val Phe Gly Asn Val Leu Val Ile Thr
   65                  70                  75                  80
   Ala Ile Ala Lys Phe Glu Arg Leu Gln Thr Val Thr Asn Tyr Phe Ile
                   85                  90                  95
   Thr Ser Leu Ala Cys Ala Asp Leu Val Met Gly Leu Ala Val Val Pro
               100                 105                 110
   Phe Gly Ala Ala His Ile Leu Met Lys Trp Thr Phe Gly Asn Phe Trp
           115                 120                 125
   Cys Glu Phe Trp Thr Ser Ile Asp Val Leu Cys Val Thr Ala Ser Ile
       130                 135                 140
   Trp Thr Leu Cys Val Ile Ala Val Asp Arg Tyr Phe Ala Ile Thr Ser
   145                 150                 155                 160
   Pro Phe Lys Tyr Gln Ser Leu Leu Thr Lys Asn Lys Ala Arg Val Ile
                   165                 170                 175
   Ile Leu Met Val Trp Ile Val Ser Gly Leu Thr Ser Phe Leu Pro Ile
               180                 185                 190
   Gln Met His Trp Tyr Arg Ala Thr His Gln Glu Ala Ile Asn Cys Tyr
           195                 200                 205
   Ala Glu Glu Thr Cys Cys Asp Phe Phe Thr Asn Gln Ala Tyr Ala Ile
       210                 215                 220
   Ala Ser Ser Ile Val Ser Phe Tyr Val Pro Leu Val Ile Met Val Phe
   225                 230                 235                 240
   Val Tyr Ser Arg Val Phe Gln Glu Ala Lys Arg Gln Leu Ala Asp Leu
                   245                 250                 255
   Glu Asp Asn Trp Glu Thr Leu Asn Asp Asn Leu Lys Val Ile Glu Lys
   260 265 270
   Ala Asp Asn Ala Ala Gln Val Lys Asp Ala Leu Thr Lys Met Arg Ala
   275 280 285
   Ala Ala Leu Asp Ala Gln Lys Ala Thr Pro Pro Lys Leu Glu Asp Lys
   290 295 300
   Ser Pro Asp Ser Pro Glu Met Lys Asp Phe Arg His Gly Phe Asp Ile
   305 310 315 320
   Leu Val Gly Gln Ile Asp Asp Ala Leu Lys Leu Ala Asn Glu Gly Lys
   325 330 335
   Val Lys Glu Ala Gln Ala Ala Ala Glu Gln Leu Lys Thr Thr Arg Asn
   340 345 350
   Ala Tyr Ile Gln Lys Tyr Leu Lys Phe Cys Leu Lys Glu His Lys Ala
   355 360 365
   Leu Lys Thr Leu Gly Ile Ile Met Gly Thr Phe Thr Leu Cys Trp Leu
   370 375 380
   Pro Phe Phe Ile Val Asn Ile Val His Val Ile Gln Asp Asn Leu Ile
   385 390 395 400
   Arg Lys Glu Val Tyr Ile Leu Leu Asn Trp Ile Gly Tyr Val Asn Ser
   405 410 415
   Gly Phe Asn Pro Leu Ile Tyr Cys Arg Ser Pro Asp Phe Arg Ile Ala
   420 425 430
   Phe Gln Glu Leu Leu Cys Leu Arg Arg Ser Ser Leu Lys His His His
   435 440 445
   His His His His His His His
   450 455
<210> 11
   <211> 403
   <212> PRT
   <213> Homo sapiens
<400> 11
   Met Lys Thr Ile Ile Ala Leu Ser Tyr Ile Phe Cys Leu Val Phe Ala
   1 5 10 15
   Asp Tyr Lys Asp Asp Asp Asp Ala Met Gly Gln Pro Gly Asn Gly Ser
               20                  25                  30
   Ala Phe Leu Leu Ala Pro Asn Arg Ser His Ala Pro Asp His Asp Val
           35                  40                  45
   Thr Gln Gln Arg Asp Glu Val Trp Val Val Gly Met Gly Ile Val Met
       50                  55                  60
   Ser Leu Ile Val Leu Ala Ile Val Phe Gly Asn Val Leu Val Ile Thr
   65                  70                  75                  80
   Ala Ile Ala Lys Phe Glu Arg Leu Gln Thr Val Thr Asn Tyr Phe Ile
                   85                  90                  95
   Thr Ser Leu Ala Cys Ala Asp Leu Val Met Gly Leu Ala Val Val Pro
               100                 105                 110
   Phe Gly Ala Ala His Ile Leu Met Lys Met Trp Thr Phe Gly Asn Phe
           115                 120                 125
   Trp Cys Glu Phe Trp Thr Ser Ile Asp Val Leu Cys Val Thr Ala Ser
       130                 135                 140
   Ile Trp Thr Leu Cys Val Ile Ala Val Asp Arg Tyr Phe Ala Ile Thr
   145                 150                 155                 160
   Ser Pro Phe Lys Tyr Gln Ser Leu Leu Thr Lys Asn Lys Ala Arg Val
                   165                 170                 175
   Ile Ile Leu Met Val Trp Ile Val Ser Gly Leu Thr Ser Phe Leu Pro
               180                 185                 190
   Ile Met His Trp Tyr Arg Ala Thr His Gln Glu Ala Ile Asn Cys Tyr
           195                 200                 205
   Ala Glu Glu Thr Cys Cys Asp Phe Phe Thr Asn Gln Ala Tyr Ala Ile
       210                 215                 220
   Ala Ser Ser Ile Val Ser Phe Tyr Val Pro Leu Val Ile Met Val Phe
   225                 230                 235                 240
   Val Tyr Ser Arg Val Phe Gln Glu Ala Lys Arg Gln Leu Met Lys Lys
                   245                 250                 255
   Tyr Thr Cys Thr Val Cys Gly Tyr Ile Tyr Asn Pro Glu Asp Gly Asp
               260                 265                 270
   Pro Asp Asn Gly Val Asn Pro Gly Thr Asp Phe Lys Asp Ile Pro Asp
           275                 280                 285
   Asp Trp Val Cys Pro Leu Cys Gly Val Gly Lys Asp Gln Phe Glu Glu
       290                 295                 300
   Val Glu Glu Lys Phe Cys Leu Lys Glu His Lys Ala Leu Lys Thr Leu
   305                 310                 315                 320
   Gly Ile Ile Met Gly Thr Phe Thr Leu Cys Trp Leu Pro Phe Phe Ile
                   325                 330                 335
   Val Asn Ile Val His Val Ile Gln Asp Asn Leu Ile Arg Lys Glu Val
               340                 345                 350
   Tyr Ile Leu Leu Asn Trp Ile Gly Tyr Val Asn Ser Gly Phe Asn Pro
           355                 360                 365
   Leu Ile Tyr Cys Arg Ser Pro Asp Phe Arg Ile Ala Phe Gln Glu Leu
       370                 375                 380
   Leu Cys Leu Arg Arg Ser Ser Leu Lys His His His His His His His
   385                 390                 395                 400
   His His His
<210> 12
   <211> 535
   <212> PRT
   <213> Homo sapiens
<400> 12
<210> 13
   <211> 497
   <212> PRT
   <213> Homo sapiens
<400> 13